(19) Europäisches Patentamt
European Patent Office
Office européen des brevets

(11) EP 4 617 372 A1

(12) **EUROPEAN PATENT APPLICATION**

(43) Date of publication:
**17.09.2025 Bulletin 2025/38**

(21) Application number: **24162781.9**

(22) Date of filing: **11.03.2024**

(51) International Patent Classification (IPC):
***C12Q 1/00*** *(2006.01)* ***C12Q 1/26*** *(2006.01)*
***B82Y 15/00*** *(2011.01)*

(52) Cooperative Patent Classification (CPC):
**C12Q 1/006; C12Q 1/26;** B82Y 15/00

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **ECOLE POLYTECHNIQUE FEDERALE DE LAUSANNE (EPFL)**
**1015 Lausanne (CH)**

(72) Inventors:
- **WANG, Hanxuan**
  **CH-1015 Lausanne (CH)**
- **BOGHOSSIAN, Ardemis Anoush**
  **1015 Lausanne (CH)**

(74) Representative: **Marks & Clerk LLP**
**15 Fetter Lane**
**London EC4A 1BW (GB)**

(54) **SWCNT GLUCOSE BIOSENSOR**

(57) An optical biosensor comprising a single-walled carbon nanotube (SWCNT) and an analyte-binding protein, wherein said analyte-binding protein is covalently conjugated to said SWCNT. Such a biosensor e.g. with glucose oxidase as binding protein, exhibits a change in optical properties when binding the analyte, allowing for its monitoring, detection and quantitation

Figure 1

a)

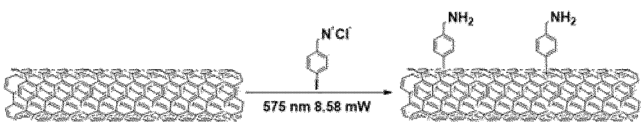


1 SWCNTs in 1% SDS

2

EP 4 617 372 A1

## Description

INTRODUCTION

The present invention relates to an optical biosensor for an analyte, to a method of preparing the optical biosensor, and to uses of the optical biosensor.

BACKGROUND

There is a frequent desire to monitor an analyte in a sample. Glucose, for example, plays a crucial role in biological systems, as it is considered to be the key molecule in cell metabolism. Glucose is involved in various pathophysiological processes and is fundamental to identifying certain health-related conditions. Testing and monitoring glucose levels is therefore very useful when, for example, seeking to control diabetes, as well as when performing cell culture monitoring and food composition analyses.

The progression of new technologies has seen measurement techniques for analyte levels, such as glucose levels, become easier to use and more convenient. Most of these contemporary techniques use the electrochemical method, in which the sensors are expensive and not reusable. For example, the typical method to achieve the level of blood glucose is by fingerstick and then testing using a specific reader. While this method can rapidly get a result, it can only be done at a single time point. For long-term and real-time monitoring, a new generation of glucose sensors is needed.

With excellent optical properties, SWCNTs show great potential in sensing applications. Laser excitations see SWCNTs emit fluorescence located at the second near-infrared (NIR-II) window, varying with the chirality and the environment of the SWCNTs. Emission wavelengths of SWCNTs do not overlap with the maximum absorption wavelengths of hemoglobin, water, or other components distributed in blood and other biofluids and tissue. Therefore, the fluorescence signal of SWCNTs could penetrate biological samples with high optical stability. These advantages provide a genuine possibility to design new optical biosensors for analytes, in particular for glucose sensing.

To achieve a reliable fluorescence signal, a good suspension of SWCNTs is required. SWCNTs have a very low solubility in water, and surfactants such as sodium dodecyl sulfate (SDS), sodium cholate (SC) and sodium dodecyl benzene sulfonate (SDBS) are often used to suspend SWCNTs. SWCNTs can also be conjugated with biomolecules that can provide specific affinity toward analytes. DNA sequences, RNA sequences, proteins, polysaccharides and lipids can be immobilized on SWCNTs' surface for further sensing applications. For example, a collection of ssDNA sequences wrapped on SWCNTs was found to identify several target molecules such as neurotransmitters, tumor markers, and drug molecules. By monitoring the modulation of the NIR fluorescent spectra in response to the analytes, these ssDNA-SWCNTs could be applied as convenient biosensors for biological and pharmaceutical detections.

In addition to nucleotides, proteins or peptides can also be used to suspend SWCNTs. For example, Vitalijs Zubkovs *et al.* (V. Zubkovs, N. Schuergers, B. Lambert, E. Ahunbay and A. A. Boghossian, 2017, **13,** 1701654) developed a non-covalent glucose oxidase (GOx)-based biosensor for glucose monitoring. Compared to DNA or RNA sequences, proteins have higher binding specificity toward the target ligand, with ligands largely recognizing specific protein biding pockets. The interaction may be based on Van der Waals forces, hydrophobic interactions, configuration changes, and/or energy transfers.

Previous work has focused on the non-covalent immobilization of enzymes on SWCNTs - see for example WO2020/254336. Although the non-covalent conjugates are easy to prepare, these conjugates are extremely heterogeneous.

There have been several methods for covalent conjugation of proteins on carbon materials; however, for sensing applications, the maintenance of the NIR signal must be considered. With sp3 defect chemistry on SWCNTs, the conjugated structure and original length of SWCNTs can be maximally retained, which is crucial for the fluorescence signal. Such techniques allow the introduction of functional handles to the SWCNTs. With these handles on functionalised SWCNTs, conjugation with proteins can be achieved. This new generation of SWCNT chemistry provides the possibility to design covalent SWCNT-based sensors.

SUMMARY OF THE INVENTION

Viewed from a first aspect, the present invention provides an optical biosensor for an analyte, the biosensor comprising

(a) a single-walled carbon nanotube (SWCNT); and
(b) an analyte-binding protein

wherein said analyte-binding protein is covalently conjugated to said SWCNT.

Viewed from another aspect, the present invention provides a method of preparing an optical biosensor as hereinbefore described, the method comprising covalently conjugating an analyte-binding protein to an SWCNT.

Viewed from another aspect, the present invention provides use of an optical biosensor as hereinbefore described to monitor an analyte in a sample.

Viewed from another aspect, the present invention provides use of an optical biosensor as hereinbefore described as a biosensor for an analyte.

Viewed from another aspect, the present invention provides use of an optical biosensor as hereinbefore described in the monitoring, detection and/or quantification of an analyte in a sample.

Viewed from another aspect, the present invention provides a method of monitoring, quantifying, and/or detecting an analyte, preferably a sugar, in a sample, the method comprising:

contacting an optical biosensor as hereinbefore described to a sample comprising an analyte, preferably a sugar;
monitoring a change in optical properties of the biosensor; and optionally
correlating the change in optical properties with an analyte level, preferably a sugar level.

Viewed from another aspect, the present invention provides a composition, preferably a suspension, comprising an optical biosensor as hereinbefore described, a solvent, and, optionally, an analyte.

Viewed from another aspect, the present invention provides a method of obtaining immobilisation, preferably structured immobilisation, of a sugar-binding protein, preferably a glucose-binding protein, on an SWCNT, comprising covalently conjugating the sugar-binding protein to the SWCNT. The method preferably comprises:

a) functionalising an SWCNT to introduce a covalent handle; and
b) covalently conjugating the sugar-binding protein to the functionalised SWCNT prepared in step (a) *via* the covalent handle.

Viewed from another aspect, the present invention provides a covalent conjugate comprising:

(a) a single-walled carbon nanotube (SWCNT); and
(b) a sugar-binding protein

wherein said sugar-binding protein is covalently conjugated to said SWCNT.

DEFINITIONS

As used herein, the term "conjugate" refers to a product where different component parts are linked or bonded together, optionally *via* one or more linkers. As used herein, the term "covalent conjugate" refers to a conjugate wherein the component parts are linked together covalently, optionally *via* one or more linkers. The optical biosensors of the present invention are covalent conjugates.

As used herein, the term "linker" refers to the part of a conjugate which links together certain different component parts of the conjugate. In a covalent conjugate, a linker will form covalent bonds to each of the components being linked.

As used herein, the term "covalent handle" refers to a functionality on one component which is capable of forming one or more covalent bonds to another component. Having formed such a covalent bond, the reacted covalent handle can be considered to form a linker, or part of a linker, between the two components. The terms "covalent handle" and "linker" are thus inter-linked, and a linker may be derived from a covalent handle.

As used herein, "E11" and "E11*" refer to characteristic peaks in the fluorescence spectrum of an SWCNT which will be known to the skilled person.

As used herein, the term "biosensor" refers to a product capable of exhibiting a response in the presence of an analyte. Preferred analytes are described below, which includes sugars, a preferred example of which is glucose.

As used herein, the term "optical biosensor" refers to a biosensor capable of exhibiting an optical response in the presence of an analyte. Examples of optical responses include a change in fluorescence properties, as described below.

As used herein, the term "sp3 defect" refers to a carbon atom of an SWCNT which has sp3 hybridisation, rather than forming part of the delocalised sp2 system of the SWCNT.

In any embodiment of the present invention, optional substituents for aryl rings may preferably include alkyl groups, for example C1-C9 alkyl groups, alkoxy groups, nitro groups, and halides. Substitution may be on any ring position, and any substituents may be branched or unbranched.

DETAILED DESCRIPTION OF THE INVENTION

The present invention provides an optical biosensor for an analyte, the biosensor comprising:

(a) a single-walled carbon nanotube (SWCNT); and
(b) an analyte-binding protein

wherein said analyte-binding protein is covalently conjugated to said SWCNT.

Preferably, said optical biosensor is for a sugar and said analyte-binding protein is a sugar-binding protein. In other words, the present invention preferably provides an optical biosensor for a sugar, the biosensor comprising:

(a) a single-walled carbon nanotube (SWCNT); and
(b) a sugar-binding protein

wherein said sugar-binding protein is covalently conjugated to said SWCNT.

Preferably, said optical biosensor is for glucose and said analyte-binding protein is a glucose-binding protein, preferably glucose oxidase. In other words, the present invention preferably provides an optical biosensor for glucose, the biosensor comprising:

(a) a single-walled carbon nanotube (SWCNT); and
(b) a glucose-binding protein, preferably glucose oxidase

wherein said glucose-binding protein is covalently conjugated to said SWCNT.

The optical biosensors of the present invention have been found to exhibit improved stability, including in harsh conditions, as compared with comparative non-covalent conjugates, making them better suited for application in complex, e.g. physiological, environments. The optical biosensors of the present invention have also been found to show strong sensitivity to a target analyte, superior to that of comparative non-covalent conjugates. The optical biosensors of the present invention are therefore more effective biosensors. The optical biosensors of the present invention could also be prepared using facile reactions, which it was found could be easily monitored. Overall, the optical biosensors of the present invention are therefore highly suited for exploitation as stable and sensitive biosensors.

*SWCNT*

The optical biosensor of the present invention comprises a single-walled carbon nanotube (SWCNT). As previously described, SWCNTs exhibit desirable optical, especially fluorescence, properties. Employing an SWCNT in the optical biosensor of the present invention may therefore improve its suitability as an optical biosensor. It was found that the optical properties of the SWCNT in the optical biosensor of the present invention could advantageously be exploited in the monitoring of an analyte, as well as in the monitoring of the reactions used to prepare the optical biosensor.

The properties of the SWCNT need not be particularly limited. For example, an SWCNT of any chirality may be employed, though a preferred chirality is (6,5). Similarly, an SWCNT of any length or diameter may be employed. However, a preferred diameter is 0.5 to 2.0 nm, more preferably 0.6 to 1.75 nm, still more preferably 0.7 to 1.5 nm. Preferred lengths may be up to 1 mm, more preferably up to 0.8 mm, for example between 0.2 to 1 mm, more preferably 0.35 to 0.8 mm. With a longer SWCNT, it may be possible to covalently conjugate more analyte-binding protein thereto, which may advantageously improve the sensitivity of the conjugate to an analyte, which may make the covalent conjugate better suited as a biosensor. Longer SWCNTs may also have higher quantum yield, which may improve the sensitivity of the optical biosensor.

*Protein*

The optical biosensor of the present invention comprises an analyte-binding protein. It will be understood that employing an analyte-binding protein may make the optical biosensor suitable for use as a biosensor for the particular analyte which is bound by that protein. In other words, in the present invention, an optical biosensor for an analyte comprises a protein capable of binding said analyte. For example, an optical biosensor comprising a glucose-binding protein may be suitable for use as an optical biosensor for glucose.

The analyte-binding protein may be a wild-type protein or a protein with one or more mutations, which may be artificial mutations. Preferably, the analyte-binding protein is an enzyme.

Preferably, the analyte is a biomolecule. Preferred analytes are selected from metal ions with biological functions (such as $Zn^{2+}$, $Mg^{2+}$, $Ca^{2+}$), proteins, lipids, antigens, nucleotides, nucleosides, nucleic acids, amino acids, vitamins,

hormones, fatty acids, glycolipids, hormones, neurotransmitters, metabolites, polysaccharides, and sugars. Particularly preferred analytes are sugars, more preferably monosaccharides or disaccharides, preferred examples of which include but are not limited to fructose, galactose, glucose, lactose, maltose, ribose, deoxyribose, and sucrose. A particularly preferred analyte is glucose. Preferably, the analyte-binding protein is therefore a protein which can bind the aforementioned analytes.

In all aspects of the present invention, the analyte-binding protein is preferably a sugar-binding protein, more preferably a glucose-binding protein. Employing a sugar-binding protein may make the optical biosensor suitable for use as a biosensor for a sugar. In other words, the analyte is preferably a sugar. A preferred sugar is glucose. Employing a glucose-binding protein may make the optical biosensor suitable for use as a biosensor for glucose. Preferred sugar-binding proteins are glucose oxidase, concanavalin A, and glucose binding protein. Glucose oxidase is a particularly preferred analyte-binding protein.

The analyte-binding protein is covalently conjugated to the SWCNT. Said another way, the analyte-binding protein can be considered to be immobilised on the SWCNT, more specifically through covalent conjugation. The optical biosensor of the present invention preferably comprises a plurality of analyte-binding proteins each covalently conjugated to the SWCNT. The presence of a plurality of, i.e. more than one, analyte-binding protein may advantageously improve the sensitivity of the conjugate to the analyte, which may make the optical biosensor better suited as a biosensor.

Preferably, the analyte-binding protein is covalently conjugated *via* its N-terminus, preferably *via* its N-terminal $-NH_2$ group, or *via* its C-terminus, preferably *via* its C-terminal -COOH group. Alternatively, the analyte-binding protein may preferably be covalently conjugated *via* an internal amino acid residue, preferably *via* a nucleophilic or electrophilic group on an internal amino acid residue, more preferably *via* an $-NH_2$ or - COOH group on an internal amino acid residue.

*Covalent Conjugation*

In the optical biosensor of the present invention, the analyte-binding protein is covalently conjugated to the SWCNT. In other words, the analyte-binding protein and the SWCNT are linked by covalent bonds. The optical biosensors of the present invention can therefore be termed covalent conjugates. It has been found that the covalent nature of the conjugates of the present invention leads to an improved stability as compared with comparative non-covalent conjugates. This may make the optical biosensor of the present invention better suited for use - e.g. for use as a biosensor - in harsh or complex environments, such as physiological environments. Preferably, the analyte-binding protein is not conjugated to the SWCNT *via* non-covalent means.

The analyte-binding protein is preferably covalently conjugated to the SWCNT *via* a linker. In other words, the optical biosensor preferably comprises:

(a) a single-walled carbon nanotube (SWCNT);
(b) an analyte-binding protein; and
(c) a linker,

wherein said analyte-binding protein is covalently conjugated to said SWCNT *via* said linker.

The linker preferably forms a covalent bond to the SWCNT and a covalent bond to the analyte-binding protein, thus enabling the analyte-binding protein to be covalently conjugated to the SWCNT *via* the linker. The optical biosensor of the present invention preferably has the structure:

SWCNT- X - p

wherein

SWCNT is a single-walled carbon nanotube;
X is a linker;
p is an analyte-binding protein; and
- are covalent bonds.

As noted, the linker and the SWCNT are preferably joined by a covalent bond. The SWCNT can be considered to be functionalised with the linker. Preferably, the linker is at an sp3 defect on the SWCNT. Said another way, the linker is preferably covalently bonded to the SWCNT *via* sp3 defect chemistry. As described in the Examples, the use of sp3 defect chemistry may advantageously allow the structure and/or optical activity of the SWCNT to be maintained, despite the presence of a covalently-bonded linker.

As noted, the linker and the analyte-binding protein are preferably joined by a covalent bond. Preferably, the analyte-binding protein and the linker are linked by a covalent bond between an electrophile-derived group on the linker

and a nucleophile-derived group on the analyte-binding protein. A nucleophile-derived group on the analyte-binding protein may preferably be a nucleophile-derived group at the N-terminus of the analyte-binding protein, for example derived from the N-terminal -$NH_2$ group of the protein. Alternatively, a nucleophile-derived group on the analyte-binding protein may be a nucleophile-derived group on an internal amino acid residue, for example derived from the side groups of nucleophilic amino acids, preferred examples being tyrosine, lysine, serine, threonine, cysteine, or non-natural nucleophilic amino acids. Any such amino acids may be present in the wild-type sequence of the analyte-binding protein, or may be present as artificial mutations.

Alternatively, the analyte-binding protein and the linker are preferably linked *via* a covalent bond between a nucleophile-derived group on the linker and an electrophile-derived group on the analyte-binding protein. An electrophile-derived group on the analyte-binding protein may preferably be an electrophile-derived group at the C-terminus of the analyte-binding protein, for example derived from the C-terminal -COOH group of the protein. Alternatively, an electrophile-derived group on the analyte-binding protein may be an electrophile-derived group on an internal amino acid residue, for example derived from the side groups of electrophilic amino acids, preferred examples being aspartic acid, glutamic acid, or non-natural electrophilic amino acids. Any such amino acids may be present in the wild-type sequence of the analyte-binding protein, or may be present as artificial mutations.

Preferably, the analyte-binding protein and the linker are covalently linked *via* a carbonyl-containing (e.g. an ester, an amide, or a thioester) bond, preferably an amide bond. Such a bond may be derived from the nucleophilic and electrophilic groups discussed above.

*Linker*

As previously described, the analyte-binding protein is preferably covalently conjugated to the SWCNT *via* a linker. The exact structure of the linker is not particularly limited. The linker may preferably be derived from a covalent handle, preferred examples of which are discussed later.

Preferably, the linker comprises a nucleophile-derived group or an electrophile-derived group. As noted above, preferably such a group forms a covalent bond to an electrophile-derived group or a nucleophile-derived group on the analyte-binding protein, respectively.

Preferably, the linker comprises a nucleophile-derived group, preferred examples including an amine-derived group, preferably an aryl amine-derived group, more preferably an aniline-derived or benzylamine-derived group. Preferably, the linker comprises an electrophile-derived group, preferred examples including a carboxylic acid-derived or ester-derived group, preferably an aryl acid-derived or aryl ester-derived group, more preferably a benzoic acid-derived or benzoic ester-derived group.

Preferably the linker comprises a group selected from the following:

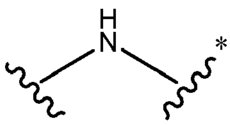 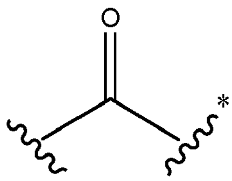

wherein wavy bonds marked by a * denote a preferred point of attachment to the analyte-binding protein. Preferably the unmarked wavy bonds denote a point of attachment to the remainder of the linker.

Preferably, the linker comprises (e.g. consists of) a group selected from the following:

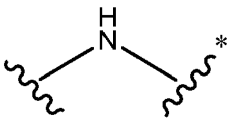
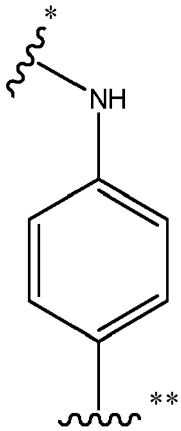
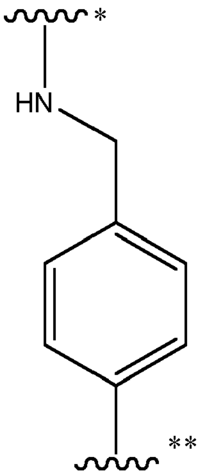
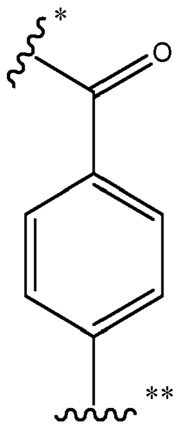
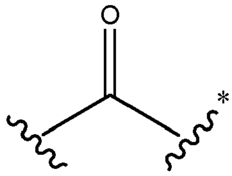

wherein the aryl rings are optionally substituted. Wavy bonds marked by a * denote a preferred point of attachment to the analyte-binding protein. Wavy bonds marked by a ** denote a preferred point of attachment to the SWCNT.

Preferably, the linker consists of a structure selected from the following:

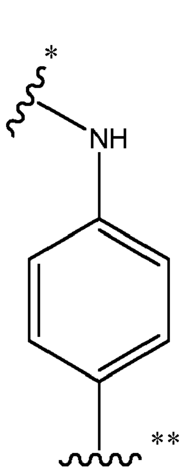
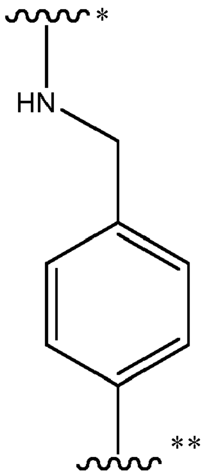
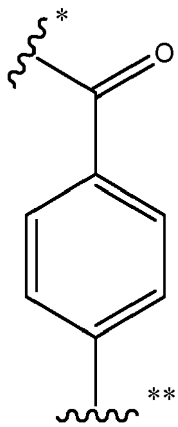

wherein the aryl rings are optionally substituted. Wavy bonds marked by a * denote a point of attachment to the analyte-binding protein. Wavy bonds marked by a ** denote a point of attachment to the SWCNT.

Preferably, the optical biosensor has the structure:

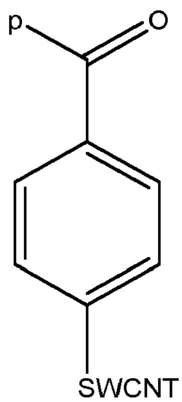

wherein

SWCNT is a single-walled carbon nanotube;
p is an analyte-binding protein;

- are covalent bonds; and
the aryl ring is optionally substituted.

Preferably, p and the linker are joined by an amide bond, preferably via a $-NH_2$-derived group on p, more preferably derived from the N-terminal $-NH_2$ of p.

Preferably, the optical biosensor has a structure selected from the following:

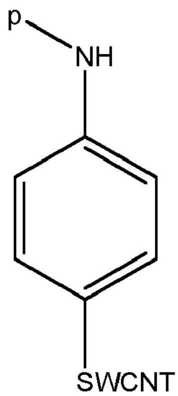
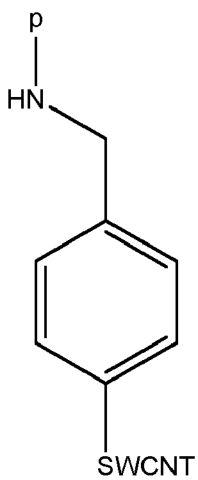

wherein

SWCNT is a single-walled carbon nanotube;
p is an analyte-binding protein;
- are covalent bonds; and
the aryl ring is optionally substituted.

Preferably, p and the linker are joined by an amide bond, preferably via a -COOH-derived group on p, more preferably derived from the C-terminal -COOH of p.

Accordingly, the optical biosensor preferably has a structure selected from the following:

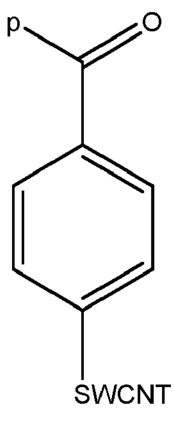
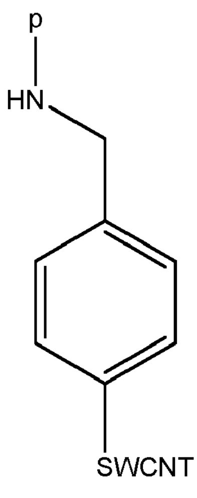
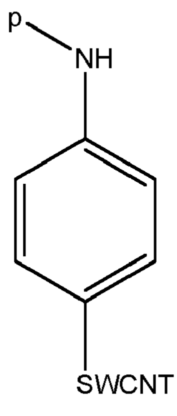

, more preferably selected from the following:

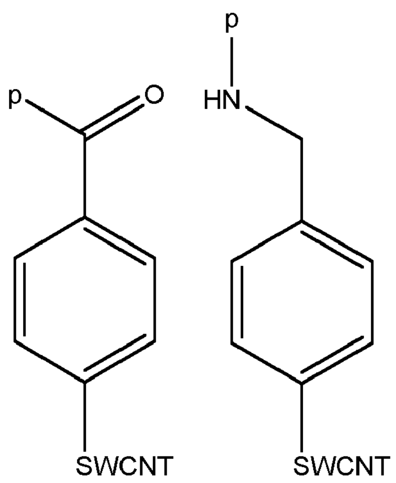

wherein

SWCNT is a single-walled carbon nanotube;
p is an analyte-binding protein, preferably a sugar-binding protein;
- are covalent bonds; and
the aryl ring is optionally substituted,
and preferably p and the linker are joined by an amide bond, preferably via a $-NH_2$-derived group on p, more preferably derived from the N-terminal $-NH_2$ of p, or via a - COOH-derived group on p, more preferably derived from the C-terminal -COOH of p.

In any embodiment of the present invention, optional substituents for aryl rings may preferably include alkyl groups, for example C1-C9 alkyl groups, alkoxy groups, nitro groups, and halides. Substitution may be on any ring position, and any substituents may be branched or unbranched.

*Properties*

As previously mentioned, the optical biosensor of the present invention can be termed a covalent conjugate. It has been found that the optical biosensor of the present invention shows improved sensitivity towards an analyte compared to non-covalent conjugates, making it well suited for use as a biosensor.

It will be understood that the optical biosensor of the present invention is suitable for use as a biosensor for an analyte. The analyte in question is determined by the analyte which may be bound by the analyte-binding protein. For example, an optical biosensor comprising a glucose-binding protein may be suitable for use as an optical biosensor for glucose.

The optical biosensor of the present invention is preferably a biosensor for a sugar, more preferably a biosensor for glucose. Said another way, the optical biosensor of the present invention is preferably suitable for use as a biosensor for a sugar, more preferably as a biosensor for glucose.

Preferably, the optical biosensor exhibits a change in optical properties, preferably a change in fluorescence properties, when binding an analyte, preferably a sugar, more preferably glucose. It will be understood that the binding of an analyte is facilitated by the analyte-binding protein. Such a change in properties can be used as the basis of biosensing applications. Preferably, such a change in optical properties is reversible on removal of the analyte. Preferably, fluorescence properties are properties of the E11* or E11 fluorescence peaks.

Preferably, the optical biosensor exhibits a change in fluorescence on binding an analyte, preferably a sugar, more preferably glucose. In other words, preferably the optical biosensor is a fluorescent biosensor, or a fluorescence-based biosensor. Possible changes in fluorescence include, but are not limited to, changes (e.g. increases or decreases, preferably increases) of fluorescence intensity (e.g. changes in intensity of one or more fluorescence peaks); shifts in the wavelengths of one or more fluorescence peaks (e.g. shifts in the wavelength of fluorescence emission); and/or a changing ratio between the intensity of two or more fluorescence peaks. Preferred fluorescence peaks are the $E_{11}$ and $E_{11}$* peaks. It is particularly preferred that the optical biosensor exhibits a change, preferably an increase, in fluorescence intensity, preferably of the $E_{11}$ and/or $E_{11}$* peaks, on binding an analyte, preferably a sugar, more preferably glucose.

Preferably, the change in optical properties, preferably a change in fluorescence properties, more preferably an increase in fluorescence intensity, is in the presence of a certain concentration of analyte, e.g. 20 mM analyte, as compared with the property in the presence of a lower concentration of analyte or, preferably, in the presence of distilled water.

Preferably, the optical biosensor exhibits a change in fluorescence intensity, preferably an increase in fluorescence intensity, as judged according to the following formula:

$$I = \frac{I_1 - I_0}{I_0}$$

wherein *I* represents normalized intensity, $I_1$ represents the intensity of the $E_{11}$ or $E_{11}$* peak on addition of a certain concentration of analyte (e.g. 20 mM analyte addition), and $I_0$ represents the intensity on the addition of deionized water. Preferably, the optical biosensor has an *I* value of greater than 20 %, more preferably greater than 30 %, still more preferably greater than 40 %, still more preferably greater than about 50 %.

Preferably, the optical biosensor has a Zeta potential, preferably as measured in the Examples, which is more positive than -20 mV, more preferably more positive than -15 mV, still more preferably more positive than about -10 mV compared to the SWCNTs *per* se. Such a Zeta potential may be indicative of superior loading or configuration of the analyte-binding protein on the SWCNT.

*Preferred embodiments*

Some preferred optical biosensors of the present invention are as follows.

An optical biosensor for a sugar, the biosensor comprising:

(a) a single-walled carbon nanotube (SWCNT); and
(b) a sugar-binding protein

wherein said sugar-binding protein is covalently conjugated to said SWCNT.

An optical biosensor for glucose, the biosensor comprising:

(a) a single-walled carbon nanotube (SWCNT); and
(b) a glucose-binding protein, preferably glucose oxidase

wherein said glucose-binding protein is covalently conjugated to said SWCNT.

An optical biosensor for a sugar, the biosensor comprising:

(a) a single-walled carbon nanotube (SWCNT); and
(b) a sugar-binding protein

wherein said glucose-binding protein is covalently conjugated to said SWCNT *via* a linker.

An optical biosensor for glucose, the biosensor comprising:

(a) a single-walled carbon nanotube (SWCNT); and
(b) a glucose-binding protein, preferably glucose oxidase

wherein said glucose-binding protein is covalently conjugated to said SWCNT *via* a linker.

An optical biosensor for a sugar, the biosensor having the structure:

SWCNT- X - p

wherein

SWCNT is a single-walled carbon nanotube;
X is a linker;
p is a sugar-binding protein; and
- are covalent bonds.

An optical biosensor for glucose, the biosensor having the structure:

SWCNT- X - p

wherein

SWCNT is a single-walled carbon nanotube;
X is a linker;
p is glucose-binding protein, preferably glucose oxidase; and
- are covalent bonds.

An optical biosensor for a sugar, preferably for glucose, having a structure selected from:

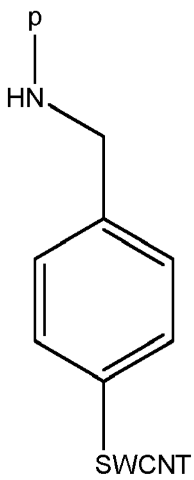
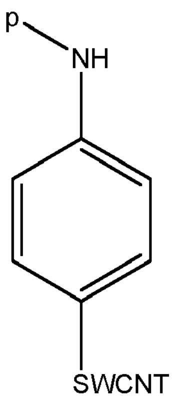
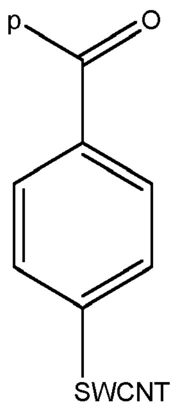

, preferably selected from:

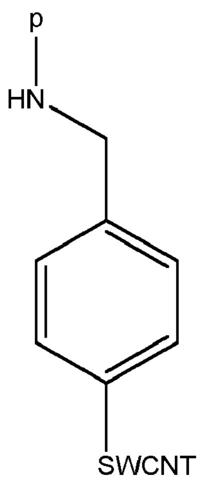
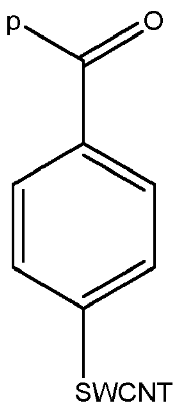

wherein

SWCNT is a single-walled carbon nanotube;
p is an sugar-binding protein, preferably a glucose-binding protein, more preferably glucose oxidase;
- are covalent bonds; and
the aryl ring is optionally substituted.

*Method*

The present invention also relates to a method of preparing an optical biosensor as hereinbefore described. The method comprises covalently conjugating an analyte-binding protein to an SWCNT. The method may preferably comprise:

a) providing a SWCNT functionalised with a covalent handle; and
b) covalently conjugating the analyte-binding protein to the functionalised SWCNT *via* the covalent handle.

Such a preferred method includes a step of providing a functionalised SWCNT. The functionalised SWCNT is functionalised with a covalent handle. The covalent handle provides chemical functionality to facilitate the covalent conjugation of the analyte-binding protein and the SWCNT.

The covalent handle and the SWCNT are preferably joined by a covalent bond. The SWCNT can be considered to be functionalised with the covalent handle. Preferably, the covalent handle is at an sp3 defect on the SWCNT. Said another way, the covalent handle is preferably covalently bonded to the SWCNT *via* sp3 defect chemistry. As described in the examples, the use of sp3 defect chemistry may advantageously allow the structure and/or optical activity of the SWCNT to be maintained, despite the presence of a covalent handle.

Preferably, the covalent handle comprises a group capable of forming a covalent bond to the analyte-binding protein. Preferred points of attachment to the analyte-binding protein are as discussed above in relation to the linker, and the group in the covalent handle can be configured (e.g. through the choice of functional groups) to permit covalent bond formation in the preferred manner, e.g. to a preferred position on the protein.

Preferably, the covalent handle comprises a nucleophilic group, preferably an amine, more preferably an aryl amine, still more preferably an aniline or a benzylamine. Preferably, the covalent handle comprises a group selected from the following:

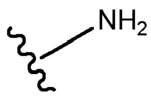
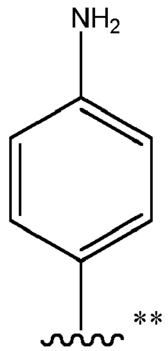
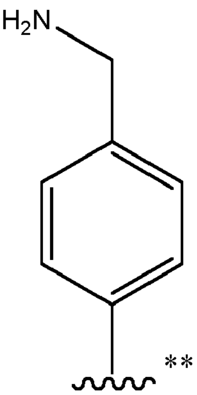

wherein the aryl rings are optionally substituted. Wavy bonds marked by ** denote a preferred point of attachment to the SWCNT.

Alternatively, the covalent handle preferably comprises an electrophilic group, preferably a carboxylic acid or ester, more preferably an aryl carboxylic acid or ester, still more preferably benzoic acid. Preferably, the covalent handle comprises a group selected from the following:

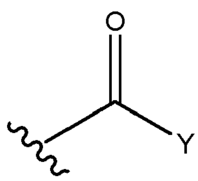
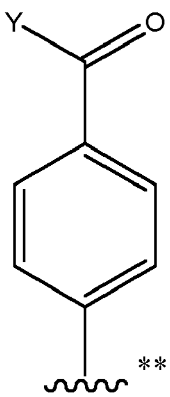

wherein the aryl rings are optionally substituted and Y is a leaving group, preferably selected from hydroxyl, halide (e.g. chloride, bromide, or iodide), or -O-alkyl, preferably wherein alkyl is branched or unbranched C1-C9 alkyl. Wavy bonds marked by ** denote a preferred point of attachment to the SWCNT.

Preferably, the covalent handle consists of a structure selected from the following:

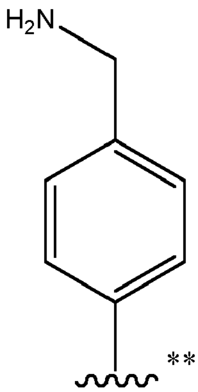
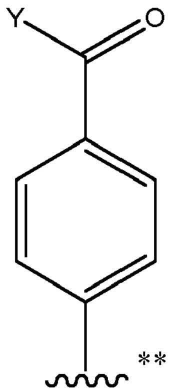
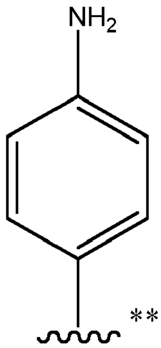

wherein the aryl rings are optionally substituted, and Y is a leaving group, preferably selected from hydroxyl, halide (e.g. chloride, bromide, or iodide), or -O-alkyl, preferably wherein alkyl is branched or unbranched C1-C9 alkyl. Wavy bonds marked by ** denote a point of attachment to the SWCNT.

Preferably, the functionalised SWCNT is SWCNT-$NH_2$ or SWCNT-COOH, as defined in the Examples.

Providing a functionalised SWCNT may preferably comprise a step of functionalising an SWCNT to introduce a covalent handle. Such a step may comprise reacting the SWCNT, preferably a suspension thereof, with a reagent configured to introduce a covalent handle. Such a reagent may have the formula LG - CH, wherein LG is a leaving group and CH is a covalent handle. When using such a reagent, the reaction will proceed *via* loss of the leaving group LG. Preferred examples for the leaving group include halide (e.g. chloride, bromide, or preferably iodide) and -$N_2^+$. Preferably, the reagent is an alkyl halide, aryl halide, or aryldiazonium salt. Particularly preferred reagents include 4-iodobenzylamine or a salt, preferably a hydrochloride, thereof, and 4-iodobenzoic acid.

Preferably, the step of reacting the SWCNT is a photocatalytic reaction, for example comprising excitation with light, for example laser light or LED light. LED light may be more suitable for large-scale reactions as compared with laser light. Preferably, the light is green light. Preferred wavelengths of light are 500 to 650 nm, more preferably 540 to 610 nm, still more preferably 550 to 600 nm, still more preferably 550 to 590 nm, most preferably about 570 nm.

As described in the examples, it has been found that a step of reacting the SWCNT can advantageously be easily monitored. Preferably, the step of reacting the SWCNT is monitored using optical properties, preferably fluorescent properties, of the SWCNT. For example, the reaction is preferably monitored using a changing ratio (e.g. intensity ratio) between the $E_{11}$* and $E_{11}$ peaks.

The method comprises a step of conjugating the analyte-binding protein to the SWCNT. Preferably, such a step is performed in water or an aqueous solvent system, e.g. aqueous DMSO. Such solvent systems have been found to be allow for better reaction performance. As described in the examples, it has been found that the step of conjugating the analyte-binding protein to the SWCNT can advantageously be easily monitored. Preferably, the step of conjugating the analyte-binding protein to the SWCNT is monitored using optical properties, preferably fluorescent properties, of the SWCNT. For example, the reaction is preferably monitored using a changing ratio (e.g. intensity ratio) between the $E_{11}$* and $E_{11}$ peaks.

Preferably the step of conjugating the analyte-binding protein to the SWCNT comprises covalently conjugating the analyte-binding protein to a functionalised SWCNT *via* the covalent handle. Preferably, such a step comprises the formation of a covalent bond between the analyte-binding protein and the covalent handle. By forming such a bond, the covalent handle becomes the linker or part of the linker between the SWCNT and the analyte-binding protein. Preferred characteristics of the bonding between this linker and the analyte-binding protein are discussed above in relation to the resulting optical biosensor.

Preferably, covalently conjugating the analyte-binding protein to the functionalised SWCNT *via* the covalent handle is by a condensation reaction. Preferably, such a step comprises pre-activating the covalent handle or the analyte-binding protein, preferably the analyte-binding protein, preferably using a coupling agent. A purpose of such a step is to improve the reactivity. Example coupling agents are known in the art, with preferred examples being EDC, NHS, or a combination thereof. Preferably such a pre-activation step is to pre-activate a carboxylic acid group on the covalent handle or on the analyte-binding protein, for example the C-terminal -COOH of the protein.

*Preferred Methods*

Some preferred methods for preparing an optical biosensor as hereinbefore described are as follows.

A method of preparing an optical biosensor for an analyte, preferably for a sugar, more preferably for glucose, the method comprising:

a) providing a SWCNT functionalised with a covalent handle; and
b) covalently conjugating an analyte-binding protein, preferably a sugar-binding protein, more preferably a glucose-binding protein, to the functionalised SWCNT *via* the covalent handle.

A method of preparing an optical biosensor for an analyte, preferably for a sugar, more preferably for glucose, the method comprising:

a) providing a SWCNT functionalised with a covalent handle; and
b) covalently conjugating an analyte-binding protein, preferably a sugar-binding-protein, more preferably a glucose-binding protein, to the functionalised SWCNT *via* the covalent handle,

wherein the covalent handle consists of a structure selected from the following:

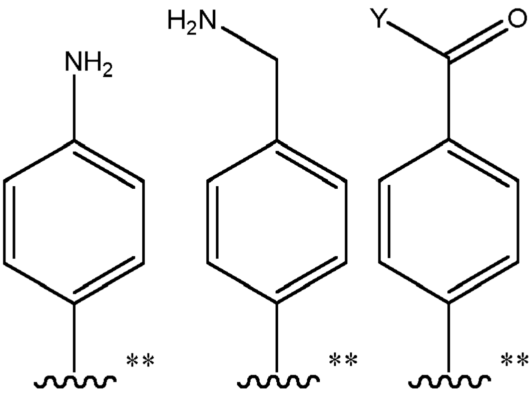

wherein the aryl rings are optionally substituted, Y is a leaving group, preferably selected from hydroxyl, halide (e.g. chloride, bromide, or iodide), or -O-alkyl, preferably wherein alkyl is branched or unbranched C1-C9 alkyl, and wavy bonds marked by ** denote a point of attachment to the SWCNT.

A method of preparing an optical biosensor for an analyte, preferably for a sugar, more preferably for glucose, the method comprising:

a) functionalising an SWCNT to introduce a covalent handle; and
b) covalently conjugating an analyte-binding protein, preferably a sugar-binding protein, more preferably a glucose-binding protein, to the functionalised SWCNT prepared in step (a) *via* the covalent handle.

A method of preparing an optical biosensor for an analyte, preferably for a sugar, more preferably for glucose the method comprising:

a) functionalising an SWCNT to introduce a covalent handle; and
b) covalently conjugating an analyte-binding protein, preferably a sugar-binding protein, more preferably a glucose-binding protein to the functionalised SWCNT prepared in step (a) *via* the covalent handle,

wherein at least one of steps (a) and (b) is monitored using optical properties, preferably fluorescent properties, of the SWCNT, preferably a ratio between $E_{11}^*$ and $E_{11}$ peaks.

A method of preparing an optical biosensor for an analyte, preferably for a sugar, more preferably for glucose, the method comprising, comprising:

a) reacting an SWCNT with a compound of formula LG - CH to prepare a functionalised SWCNT being functionalised with a covalent handle; and
b) covalently conjugating an analyte-binding protein, preferably a sugar-binding protein, more preferably a glucose-binding protein, to the functionalised SWCNT prepared in step (a) *via* the covalent handle,

wherein CH is a covalent handle and LG is a leaving group.

A method of preparing an optical biosensor for an analyte, preferably for a sugar, more preferably for glucose, the method comprising:

a) reacting an SWCNT with a compound of formula LG - CH to prepare a functionalised SWCNT being functionalised with a covalent handle; and
b) covalently conjugating an analyte-binding protein, preferably a sugar-binding protein, more preferably a glucose-binding protein, to the functionalised SWCNT prepared in step (a) *via* the covalent handle,

wherein CH is a covalent handle and LG is a leaving group,
preferably wherein LG is selected from halide or $-N_2^+$ and/or preferably wherein CH is selected from:

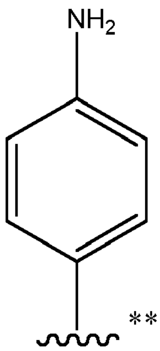
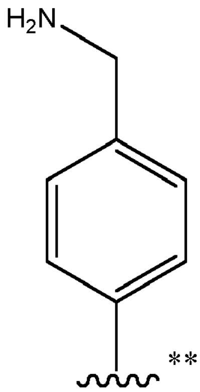
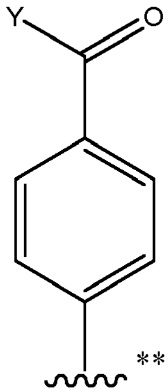

wherein the aryl rings are optionally substituted, Y is a leaving group, preferably selected from hydroxyl, halide (e.g. chloride, bromide, or iodide), or -O-alkyl, preferably wherein alkyl is branched or unbranched C1-C9 alkyl, and wavy bonds marked by ** denote a point of attachment to the SWCNT.

*Applications*

The present invention also relates to the use of the optical biosensors hereinbefore described.
For example, the present invention relates to the use of the optical biosensor as a biosensor for an analyte, preferably as a biosensor for a sugar, more preferably as a biosensor for glucose. The present invention also relates to use of the optical biosensor to monitor an analyte, preferably a sugar, more preferably glucose, in a sample and to the use of the optical biosensor in the detection and/or quantification of an analyte, preferably a sugar, more preferably glucose, in a sample. The exact analyte for the biosensor will be determined by the choice of analyte-binding protein.
The present invention also relates to a method of monitoring, quantifying, and/or detecting an analyte, preferably a sugar, more preferably glucose, in a sample, the method comprising:

contacting an optical biosensor as hereinbefore described to a sample comprising an analyte, preferably a sugar, more preferably glucose;
monitoring a change in optical properties of the conjugate; and optionally
correlating the change in optical properties with an analyte level.

Preferred optical properties are fluorescent properties, as described above in relation to the properties of the optical biosensor. As described in the Examples, it has been found that the optical biosensor of the present invention can be used as biosensor with improved stability and sensitivity.
In preferred methods and uses of the invention, the sample is pre-obtained. Methods and uses of the present invention may be *in vitro, in vivo,* or *ex vivo* methods. Preferred methods and uses of the present invention are *in vitro* or *ex vivo* methods, more preferably *in vitro.*
The present invention also relates to a composition, preferably a suspension, comprising the optical biosensor, a solvent, and, optionally, an analyte, preferably a sugar, more preferably glucose.
The present invention also relates to a method of obtaining immobilisation, preferably structured immobilisation, of a sugar-binding protein, preferably a glucose-binding protein, on an SWCNT, comprising covalently conjugating the sugar-binding protein to the SWCNT. The method preferably comprises:

a) functionalising an SWCNT to introduce a covalent handle; and
b) covalently conjugating the sugar-binding protein to the functionalised SWCNT prepared in step (a) *via* the covalent handle.

The present invention also relates to a covalent conjugate comprising:

(a) a single-walled carbon nanotube (SWCNT); and
(b) a sugar-binding protein

wherein said sugar-binding protein is covalently conjugated to said SWCNT.

Preferred embodiments for, e.g., the different components of, features of, properties of, uses of, and methods for preparing the covalent conjugate are as described above for the optical biosensors of the present invention.

The invention will now be described with reference to the following non-limiting examples and Figures, wherein:

Figure 1(a) shows the reaction between SWCNTs and 4-iodobenzylamine hydrochloride;
Figure 1(b) shows reaction monitoring by NIR-fluorescent spectra of SWCNTs;
Figure 1(c) shows a PL map before (left) and after (right) the photocatalyst reaction;
Figure 1(d) is a UV-Vis-NIR absorption of SWCNTs before reaction (SWCNTs), after reaction ($NH_2$-SWCNTs), and after washing by water and 1%SDS to remove the impurities ($NH_2$-SWCNTs-purified).;
Figure 1(e) shows the introduction of carboxylic groups on SWCNTs. Top: scheme of reactions; Bottom: Reaction monitoring by NIR-fluorescent spectra of SWCNTs;
Figure 2(a) depicts a scheme of reaction condition screening;
Figure 2(b) shows the strategy of pre-activation of the GOx with $NH_2$-SWCNTs;
Figure 2(c) shows reaction monitoring by NIR-fluorescent spectra of SWCNTs;
Figure 2(d) shows the NIR-fluorescent spectra of $NH_2$-SWCNTs and in particular that the $NH_2$-SWCNTs have a response toward EDC and cover the real shift from the reaction process;
Figure 3(a) shows FT-IR spectra of SC, non-functionalised SWCNTs in 2% SC, covalent conjugate, and pure GOx protein, respectively;
Figure 3(b) shows the zeta potential of non-functionalised SWCNTs, non-covalent conjugate comprising GOx non-covalently conjugated to non-functionalised SWCNTs, and covalent conjugate, respectively (each test was repeated 3 times and the average reported);
Figure 4 shows an aabsorption spectrum of FITC-GOx, the characteristic peaks at 280nm and 497nm belong to GOx and FITC, respectively;
Figure 5(a) shows ttreatment of conjugates before observed by microscopy;
Figure 5(b) shows comparative non-covalent conjugate comprising GOx non-covalently conjugated to non-functionalised SWCNTs loses the colocalization of SWCNTs and GOx
Figure 5(c) shows covalent conjugate maintains the colocalization of SWCNTs and GOx;
Figure 5(d) shows covalent conjugate and non-covalent conjugate after adding methanol. The covalent conjugate can still have suspension (left) and the comparative non-covalent conjugate comprising GOx non-covalently conjugated to non-functionalised SWCNTs will form the precipitate very fast after centrifuge (right);
Figure 6(a) shows the responses of different SWCNTs-based sensors. (6,5) SWCNTs, or defect (6,5) SWCNTs are used for calculation (mean, n=3). The concentration of glucose is 20 mM; and
Figure 6(b) shows the NIR-fluorescent spectra of covalent conjugate before and after adding glucose.

EXAMPLES

**Materials**

All materials were obtained from commercial sources, unless stated otherwise.

Purified SWCNTs were purchased from CHASM (SG65i, produced using CoMoCAT™ synthesis technology).

Glucose oxidase (GOx) (from Aspergillus niger) was purchased from TCI.

Bovine serum albumin (BSA) was purchased from Hello Bio™.

The following reagents were purchased from Sigma-Aldrich:

4-iodobenzylamine hydrochloride
4-iodobenzoic acid
1-Ethyl-3-(3-dimethylaminopropyl) carbodiimide (EDC)
N-Hydroxysuccinimide (NHS)
Tris(hydroxymethyl)aminomethane (Tris)
Sodium cholate (SC) hydrate.

Sodium Dodecyl Sulfate (SDS) was purchased from Carl Roth.

Fluorescein isothiocyanate (FITC) was purchased from ABCR.
PBS was purchased from Gibco™.
Amicon® Ultra devices were purchased from Merck.
Glass bottom Dishes were purchased from MatTek Life Sciences.

*Example 1: Preparation of SWCNT Suspension*

*Methodology*

The purpose of this Example was to prepare a suspension of SWCNTs.

45 mg of SWCNTs (SG65i) was mixed with 45 ml 1% SDS solution and homogenized for 30 min at 5,000 rpm (PT 1300D, Polytron). The mixture was sonicated in an ice bath for 1 hour (Q700 Sonicator, Qsonica, 10% amplitude). After sonication, the suspension was centrifuged at 10,000 RPM for 10 min at first and then at 30,000 RPM for 4 hours (Optima XPN-80, Beckman Coulter), and the supernatant was collected to remove the precipitates.

To calculate the concentration of SWCNTs, a UV-Vis-NIR spectrometer (Shimadzu 3600 Plus) was employed to measure the absorption at 739 nm, and the concentration was calculated using an extinction coefficient of 25.3 mL/(mg·cm) according to the methodology of J. Yang, Z. Zhang, D. Zhang and Y. Li, Materials Chemistry and Physics, 2013, 139, 233-240.

Example 1 thus yielded a suspension of SWCNTs which was carried forward to Example 2.

*Example 2: Preparing Functionalised SWCNTs*

*Methodology*

The purpose of this Example was to prepare functionalised SWCNT's comprising covalent handles with either $NH_2$- or COOH- functional groups, termed $NH_2$-SWCNTs and COOH-SWCNTs respectively.

The suspension prepared in Example 1 was adjusted to a concentration of 40mg/L in 1% SDS. Deionized water was added to the suspension to further dilute the SWCNTs to 10mg/L. 4-iodobenzylamine hydrochloride was dissolved in water to 50mM and 4-iodobenzoic acid was dissolved in ethanol to 50mM, respectively, for use in two separate reactions. In each case, 50 μL of the solution was added to 5 ml diluted SWCNTs suspension with stirring. Each reaction was excited by a 575 nm laser for around 48 hours (depending on the power of the laser and the amount of mixture) to form $NH_2$-SWCNTs and COOH-SWCNTs, respectively. It was also found that green LED with higher power could be applied to larger-scale preparations, in place of the laser. Each reaction was monitored by NIR signal. In each case, after the completion of the reaction, the mixture was transferred into the Amicon® Ultra device and washed with 1% SDS to remove the unreacted compounds. SDS was also exchanged to sodium cholate (SC) *via* the Amicon® Ultra device to avoid the denaturing of proteins. The concentration of functionalised SWCNTs was determined by UV-Vis-NIR spectrometer.

Example 2 thus yielded $NH_2$-SWCNTs and COOH-SWCNTs. $NH_2$-SWCNTs were carried forward to Example 3.

*Discussion*

As described above, a photocatalyst reaction with 4-iodobenzylamine hydrochloride was employed to introduce covalent handles comprising amino groups onto the SWCNTs (Fig 1a). Band gap changing led to the formation of the $E_{11}$* peak around 1150nm. The intensity of $E_{11}$* peak increased obviously with increasing SWCNT functionalisation. The ratio between $E_{11}$* and $E_{11}$ peak could thus advantageously be used to index the reaction progression (Fig 1b).

A PL (photoluminescence) map was also employed to evaluate the SWCNTs before and after the reaction (Fig 1c). From the PL-in map, it was further confirmed that the peak around 1150nm is originally from $E_{11}$ peak of (6,5) SWCNTs rather than shift from other chiralities. This means that reaction monitoring is easier due to the distinctive peak. Moreover, the same peak can also be monitored in subsequent reactions.

UV-Vis-NIR spectrometry was also employed to acquire absorption spectra (Fig 1d). The shape and position of peaks are similar in the non-functionalised and functionalised SWCNT's, which evidences that the SWCNT structure is advantageously maintained.

After the successful demonstration of the sp3 defect reaction with 4-iodobenzylamine hydrochloride, the reaction was expanded to 4-iodobenzoic acid (Fig. 1e). The intensity of the $E_{11}$* peak again increased obviously with increasing SWCNT functionalisation, meaning that the ratio between $E_{11}$* and $E_{11}$ peak could again advantageously be used to index the reaction progression.

In summary, the reactions described above can be used to successfully functionalise SWCNTs with covalent handles comprising amino groups or carboxylic groups under mild conditions and with convenient purifications. This facile reactivity is beneficial for bioconjugation chemistry on SWCNTs. The structure of the SWCNT's is also advantageously

maintained following functionalisation, as evidenced by the UV-Vis-NIR spectrometry data.

*Example 3: Non-covalent (comparative) and covalent conjugation of protein to functionalised SWCNTs*

*Methodology*

The purpose of this Example was to prepare covalent conjugates and comparative non-covalent conjugates.

Using covalent and non-covalent methods, GOx protein was loaded on the $NH_2$-SWCNTs obtained in Example 2.

For the covalent method, 20 mg GOx was dissolved in water or the specified buffer. 1 μL EDC was dissolved in water, and 5 mg NHS was dissolved in DMSO. When the sulfo-NHS (N-hydroxysulfosuccinimide) was applied, all the compounds could be dissolved in water. All the coupling reagents were added to the GOx solution for the activation. After 1 hour of mixing, the activated GOx was subject to ultrafiltration using an Amicon® Ultra device to remove excess coupling reagent. The activated GOx was then added to functionalised SWCNTs ($NH_2$-SWCNTS) for the bioconjugation reaction at room temperature. After the reaction, Amicon® Ultra devices were applied to remove the impurities to purify the conjugate.

The non-covalent conjugates were prepared under the protocol of Zubkovs, V. et al, Small 2017, 13, 1701654. GOx was directly immobilized on both non-functionalised SWCNTs from Example 1 and $NH_2$-SWCNTs obtained in Example 2by dialysis.

Example 3 thus yielded:

- a covalent conjugate comprising GOx covalently conjugated to functionalised SWCNTs *via* covalent handles;
- a comparative non-covalent conjugate comprising GOx non-covalently conjugated to functionalised $NH_2$-SWCNTs; and
- a comparative non-covalent conjugate comprising GOx non-covalently conjugated to non-functionalised SWCNTs.

*Discussion*

As described above, with $NH_2$-SWCNTs **2** in hand, covalent conjugates with $GO_x$ were synthesised.

Different combinations of coupling reagents were screened (Fig 2a), and it was found that with the presence of EDC the reaction could proceed well. However, EDC itself has an interaction with SWCNTs, which leads to a strong increase in NIR intensity. This unusual rise covered other intensity changes and peak shifts and made the reaction difficult to monitor (Fig 2d).

Preactivating GOx with EDC and NHS provides the possibility to remove the excess EDC before adding to SWCNTs, with a view to improving reaction monitoring GOx was treated with EDC and NHS in a DMSO/water cosolvent system and the formed activated ester had moderate stability for ultrafiltration. After ultrafiltration, the excess coupling reagent was removed, and the GOx with active ester terminal was mixed with $NH_2$-SWCNTs (Fig 2b).

Several buffers were screened and it was found that the ions in the buffer will affect the stability of SWCNTs and form a precipitate. The conjugation reaction was therefore judged to run better in water or in an aqueous solvent system.

The reaction was then monitored by NIR spectra (Fig 2c). It was found that the $E_{11}$ peak maintained the original intensity while the $E_{11}$* peak raised a lot. The ratio between $E_{11}$* and $E_{11}$ changed from 1.54 to 4.24; this significant change showed the reaction site is in defect positions. At the same time, redshift occurred at the $E_{11}$* peak, which is also observed in many proteins' immobilization on SWCNTs protocols. The data therefore supports the formation of a covalent bond, as well as providing for the possibility of real-time reaction monitoring.

*Example 4: Zeta potential and FT-IR Characterization of Covalent Conjugates*

*Methodology*

The purpose of this Example was to characterise the covalent conjugates and comparative non-covalent conjugates produced in Example 3.

The suspension of covalent conjugate obtained in Example 3 was diluted in PBS buffer with a concentration of around 10 mg/L. The same was done to the suspension of non-functionalised SWCNTs obtained in Example 1 to act as a control. The same was also done to a suspension of comparative non-covalent conjugate comprising GOx non-covalently conjugated to non-functionalised SWCNTs as obtained in Example 3. Zeta potential was measured on Zetasizer Nano ZS (Malvern).

Separately, the suspension of covalent conjugates obtained in Example 3 was added dropwise on glass slides and heated at 70 °C to form the dry SWCNTs film. The same was done to the suspension of SWCNTs obtained in Example 1 to act as a control. Further control groups, including sodium cholate and GOx protein, directly use the powder of the samples without further purification. All the FT-IR spectra were obtained on Spectrum 3 FT-IR spectrometer (PerkinElmer).

*Discussion*

After the synthesis of covalent and non-covalent conjugates, the properties by FT-IR and Zeta potential were further characterised, as described above.

SWCNTs themselves don't have many characteristic peaks in FT-IR, thus monitoring the characteristic peaks of wrapping material on SWCNTs becomes important (Fig 3a). It was noticed that the SWCNTs suspended in sodium cholate almost have the same spectra as pure sodium cholate powder. At the same time, it was found in the covalent conjugate that a single peak around 1600 $cm^{-1}$ disappeared and was replaced by the double peak of amide bound from protein. It is worth noting that the shape of the double peak changed a lot, indicating that the conjugate is not a simple mixture, but with a stronger covalent link which affects the spectra of GOx. At the same time, the decrease of the C-H peak at 3000 $cm^{-1}$ also shows the removal of sodium cholate.

A zeta potential analysis was also employed to evaluate the changes in SWCNTs' surface electrical properties (Fig 3b). The untreated SWCNTs suspension had a strong negative charge. With the immobilization of GOx, whether as non-covalent or covalent conjugate, the zeta potential increases a lot (i.e. becomes more positive). It was also noticed that the covalent conjugate had a much higher (i.e. more positive) zeta potential than the non-covalent conjugate. This result might be caused by the higher loading or better configuration of GOx on the the SWCNTs, which is beneficial for analyte sensing.

In summary, the characterisation analysis demonstrates the successful covalent conjugation of GOx to SWCNTs *via* the covalent handle, using the methodology described in Example 3. The FT-IR analysis evidences the covalent character of the conjugation, while the zeta potential analysis suggests superior loading or configuration of GOx on SWCNTs as compared with the comparative non-covalent conjugate.

*Example 5: Analysis of* co-localisation *of GOx and SWCNTs*

*Methodology*

The purpose of this Example was to investigate co-localisation of protein and (functionalised) SWCNT's, with a view to assessing the stability of the conjugates.

The GOx protein was first labelled by FITC by the following procedure. Dissolved 10 mg GOx in 1mL $NaHCO_3/Na_2CO_3$ buffer and 10 mg FITC in 100 μL DMSO. The solution of FITC was added to the GOx solution and the mixture was shaken at room temperature for 1h. After shaking, the mixture was stored at 4 °C overnight and purified by Amicon® Ultra devices. After the removal of free FITC, the absorption spectra of labeled GOx were obtained by Varioskan LUX Multimode Microplate Reader.

The labelled GOx was linked with SWCNTs by the covalent or non-covalent methods (comparative non-covalent conjugate comprising GOx non-covalently conjugated to non-functionalised SWCNTs) described in Example 3. All the conjugates were added to the poly-L-lysine-coated petri dishes and immobilized in the dark for 1 hour. After that, the petri dishes were washed with water and HEPES for three rounds (for the evaluation of stability, the petri dishes were washed with 5% SDS and methanol).

The co-localization of GOx and SWCNTs was identified on a custom-built microscope setup (based on the Nikon Eclipse Ti-E microscope) with oil-immersion TIRF Apo 100 x objective (N.A. 1.49, Nikon). The images were captured by InGaAs NIR camera (NIRvana 640 ST, Princeton Instruments) or EMCCD visible camera (iXon Ultra 888). All the image processing was done by Image Fiji software.

*Discussion*

As described above, to further identify the co-localisation and evaluate the stability of the conjugates, GOx was labelled with FITC to visualize the proteins (Fig 4). With the same procedure described in Example 3, both the covalent conjugate and comparative non-covalent conjugate comprising GOx non-covalently conjugated to non-functionalised SWCNTs was prepared. Petri dishes were coated with poly-L-lysine, and then the conjugate was immobilised on the surface of SWCNTs. After that, the petri dishes were washed under very harsh conditions (5% SDS and methanol) to break the nonspecific interaction between GOx and SWCNTs, and then observed by microscope (Fig 4a).

In each case, the fluorescence of both GOx and SWCNTs was stable under the treatment. In the covalent conjugate, the position of labelled GOx and SWCNTs still had a high consistency following treatment. However, the comparative non-covalent conjugate lost the co-localisation between SWCNTs and GOx (Fig 4b, 4c). The suspension of the covalent conjugate is also observed to have less precipitate than the non-covalent conjugate after adding methanol (Fig 4d), which also demonstrated that the covalent conjugate had superior stability.

In summary, the co-localisation analysis demonstrates that the covalent conjugate of the present invention had superior stability as compared with comparative non-covalent conjugates.

*Example 6: Glucose Response*

*Methodology*

The purpose of this Example was to characterise the response of conjugates to glucose, using NIR fluorescence techniques.

All the NIR spectra were obtained by a microscope setup (based on the Nikon Eclipse Ti-E microscope). To each well of a 384-well plate was added 20 μL of either covalent or non-covalent conjugates (either comparative non-covalent conjugate comprising GOx non-covalently conjugated to non-functionalised SWCNTs or comparative non-covalent conjugate comprising GOx non-covalently conjugated to functionalised $NH_2$-SWCNTs) obtained from Example 3 and either 20 μL of 20 mM glucose solution or 20 μL deionized water. The SWCNTs were excited at 570 ± 10 nm (SuperK Extreme EXR-15 and SuperK Varia, NKT Photonics) laser, and the NIR fluorescence spectra were acquired between 950 nm and 1400 nm by InGaAs NIR camera (NIRvana 640 ST, Princeton Instruments) coupled with IsoPlane SCT-320 spectrometer (Princeton Instruments).

The intensity of both $E_{11}$ and defected $E_{11}$* peaks were measured to calculate the normalized response I towards glucose:

$$I = \frac{I_1 - I_0}{I_0}$$

($I$ represents normalized intensity, $I_1$ represents the intensity of $E_{11}$ or $E_{11}$* peaks on 20 μL glucose addition, and $I_0$ represents the intensity on the addition of 20 μL deionized water.)

Measured I values are summarised below:

| Sample | Fluorescence peak being used | *I* (average of three repeats) | Standard Deviation |
|---|---|---|---|
| PBS | E11 | -8.15% | ±5% |
| Non-covalent conjugate comprising GOx non-covalently conjugated to non-functionalised SWCNTs | E11 | 19.9% | ±2.8% |
| Non-covalent conjugate comprising GOx non-covalently | E11 | 10.06% | ±2.8% |
| conjugated to functionalised $NH_2$-SWCNTs | | | |
| Non-covalent conjugate comprising GOx non-covalently conjugated to functionalised $NH_2$-SWCNTs | E11* | 4.27% | ±1.2% |
| Covalent conjugate | E11 | 54.80% | ±4.5% |
| Covalent conjugate | E11* | 47.91% | ±5.6% |

*Discussion*

As discussed above, the response of GOx-SWCNTs-based sensors toward 20 mM glucose was evaluated (Fig 6a). Comparative non-covalent conjugate comprising GOx non-covalently conjugated to non-functionalised SWCNTs shows around 20% intensity change (calculated by the equation above). This result is similar to the result from the literature, which proved the evaluation is reliable.

For comparative non-covalent conjugate comprising GOx non-covalently conjugated to functionalised $NH_2$-SWCNTs, it was found that both responses from $E_{11}$ and $E_{11}$* peaks decreased; this result may be because the functional groups affect the attachment of GOx on $NH_2$-SWCNTs, and also makes it easier to form a precipitate.

However, the response on both $E_{11}$ and $E_{11}$* peaks of the covalent conjugate become more significant and ***I*** can reach more than 50% intensity change (Fig 6b).

The greater intensity from the covalent conjugate indicates the higher GOx loading on SWCNTs, which was also proved by zeta potential. At the same time, it was also observed that the $E_{11}$ peak rises at the same level as the $E_{11}$* peak, which, without wishing to be bound by any theory, may indicate that the covalent linking improved electron and energy transfer efficiency.

In summary, the covalent conjugates of the present invention showed a response to glucose having superior intensity as compared with comparative non-covalent conjugates. Accordingly, the covalent conjugates of the present invention can be used as superior biosensors, with improved sensitivity.

## Conclusion

In summary, using sp3 defect chemistry, functionalised SWCNTs with covalent handles comprising amino and carboxylic groups were engineered. The reactions desirably proceeded with mild conditions and convenient purifications. The functional handles can be exploited for further conjugation reactions, such as covalent conjugation with GOx.

The application of the formed covalent conjugates in biosensing was demonstrated, with greater sensitivity towards a glucose analyte than comparative non-covalent conjugates. The covalent conjugate also shows superior stability under harsh conditions, which also provides the possibility for further application in complex physiological environments.

The covalent conjugates were thus shown to be particularly effective optical biosensors for a target analyte.

At the same time, the changing ratio between the $E_{11}$ and $E_{11}*$ peak was demonstrated, meaning that the described reactions can be real-time monitored by NIR spectra, which gives a new perspective on SWCNTs conjugation reaction monitoring.

REFERENCES

1. K. Zierler, 1999, **276,** E409-E426.
2. O. Schnell and E. Standi, Endocrine Practice, 2006, 12, 16-19.
3. S. Dewanjee, P. Chakraborty, H. Bhattacharya, L. Chacko, B. Singh, A. Chaudhary, K. Javvaji, S. R. Pradhan, J. Vallamkondu, A. Dey, R. S. Kalra, N. K. Jha, S. K. Jha, P. H. Reddy and R. Kandimalla, Free Radical Biology and Medicine, 2022, 193, 134-157.
4. A. L. Galant, R. C. Kaufman and J. D. Wilson, Food Chemistry, 2015, 188, 149-160.
5. M.-S. Steiner, A. Duerkop and O. S. Wolfbeis, Chemical Society Reviews, 2011, 40, 4805-4839.
6. E. S. Forzani, H. Zhang, L. A. Nagahara, I. Amlani, R. Tsui and N. Tao, Nano Letters, 2004, 4, 1785-1788.
7. L. Olansky and L. Kennedy, Diabetes Care, 2010, 33, 948-949.
8. M. El Khoury, F. Yousuf, V. Martin and R. M. Cohen, Endocrine Practice, 2008, 14, 337-339.
9. J. I. Joseph, 2021, **15,** 167-173.
10. Y. Lin, M. Bariya, H. Y. Y. Nyein, L. Kivimäki, S. Uusitalo, E. Jansson, W. Ji, Z. Yuan, T. Happonen, C. Liedert, J. Hiltunen, Z. Fan and A. Javey, 2019, **29,** 1902521.
11. J. Ackermann, J. T. Metternich, S. Herbertz and S. Kruss, 2022, **61,** e202112372.
12. A. A. Boghossian , J. Zhang , P. W. Barone, N. F. Reuel, J.-H. Kim, D. A. Heller, J.-H. Ahn, A. J. Hilmer, A. Rwei, J. R. Arkalgud, C. T. Zhang and M. S. Strano, 2011, **4**, 848-863.
13. T. V. Galassi, M. Antman-Passig, Z. Yaari, J. Jessurun, R. E. Schwartz and D. A. Heller, PLOS ONE, 2020, 15, e0226791.
14. D. Meyer, S. Telele, A. Zelená, A. J. Gillen, A. Antonucci, E. Neubert, R. Nißler, F. A. Mann, L. Erpenbeck, A. A. Boghossian, S. Köster and S. Kruss, Nanoscale, 2020, 12, 9104-9115.
15. J. Xu, R. Mueller, E. Hazelbaker, Y. Zhao, J.-C. J. Bonzongo, J. G. Clar, S. Vasenkov and K. J. Ziegler, Langmuir, 2017, 33, 5006-5014.
16. A. Hirano, T. Kameda, Y. Yomogida, M. Wada, T. Tanaka and H. Kataura, 2016, **2**, 911-920.
17. R. Haggenmueller, S. S. Rahatekar, J. A. Fagan, J. Chun, M. L. Becker, R. R. Naik, T. Krauss, L. Carlson, J. F. Kadla, P. C. Trulove, D. F. Fox, H. C. DeLong, Z. Fang, S. O. Kelley and J. W. Gilman, Langmuir, 2008, 24, 5070-5078.
18. S. Kruss, M. P. Landry, E. Vander Ende, B. M. A. Lima, N. F. Reuel, J. Zhang, J. Nelson, B. Mu, A. Hilmer and M. Strano, Journal of the American Chemical Society, 2014, 136, 713-724.
19. R. Nißler, L. Kurth, H. Li, A. Spreinat, I. Kuhlemann, B. S. Flavel and S. Kruss, Analytical Chemistry, 2021, 93, 6446-6455.
20. M. P. Landry, L. Vuković, S. Kruss, G. Bisker, A. M. Landry, S. Islam, R. Jain, K. Schulten and M. S. Strano, *The Journal of Physical Chemistry* C, 2015, **119,** 10048-10058.
21. A. Antonucci, J. Kupis-Rozmysłowicz and A. A. Boghossian, *ACS Applied Materials & Interfaces,* 2017, **9,** 11321-11331.
22. X. Zhang, L. Meng and Q. Lu, ACS Nano, 2009, 3, 3200-3206.
23. Y. Yomogida, T. Tanaka, M. Tsuzuki, X. Wei and H. Kataura, ACS Applied Nano Materials, 2020, 3, 11289-11297.
24. A. Hendler-Neumark, V. Wulf and G. Bisker, ACS Sensors, 2023, 8, 3713-3722.
25. C. Farrera, F. Torres Andón and N. Feliu, ACS Nano, 2017, 11, 10637-10643.
26. V. Zubkovs, N. Schuergers, B. Lambert, E. Ahunbay and A. A. Boghossian, 2017, **13,** 1701654.
27. M. Laskowski and M. A. Qasim, Biochimica et Biophysica Acta (BBA) - Protein Structure and Molecular Enzymology, 2000, 1477, 324-337.

28. L. J. C. Jeuken, A. K. Jones, S. K. Chapman, G. Cecchini and F. A. Armstrong, Journal of the American Chemical Society, 2002, 124, 5702-5713.
29. C. Bergeret, J. Cousseau, V. Fernandez, J.-Y. Mevellec and S. Lefrant, The Journal of Physical Chemistry C, 2008, 112, 16411-16416.
30. H. Wang and A. A. Boghossian, Materials Advances, 2023, 4, 823-834.
31. Y. Piao, B. Meany, L. R. Powell, N. Valley, H. Kwon, G. C. Schatz and Y. Wang, Nature Chemistry, 2013, 5, 840-845.
32. J. Zaumseil, 2022, **10,** 2101576.
33. T. Shiraki, Y. Miyauchi, K. Matsuda and N. Nakashima, Accounts of Chemical Research, 2020, 53, 1846-1859.
34. N. D. J. Yates, M. A. Fascione and A. Parkin, 2018, **24,** 12164-12182.
35. F. A. Mann, N. Herrmann, F. Opazo and S. Kruss, 2020, **59,** 17732-17738.
36. J. De Meutter and E. Goormaghtigh, Analytical Chemistry, 2021, 93, 3733-3741.
37. X. Zhao, D. Lu, F. Hao and R. Liu, Journal of Hazardous Materials, 2015, 292, 98-107.
38. L. Chio, R. L. Pinals, A. Murali, N. S. Goh and M. P. Landry, 2020, **30,** 1910556.
39. F. A. Mann, N. Herrmann, F. Opazo, S. Kruss, Angew. Chem. Int. Ed. 2020, 59, 17732.

## Claims

**1.** An optical biosensor for an analyte, the biosensor comprising:

(a) a single-walled carbon nanotube (SWCNT); and
(b) an analyte-binding protein

wherein said analyte-binding protein is covalently conjugated to said SWCNT.

**2.** An optical biosensor according to claim 1, wherein said optical biosensor is for a sugar and said analyte-binding protein is a sugar-binding protein.

**3.** An optical biosensor according to claim 2, wherein said optical biosensor is for glucose and said sugar-binding protein is a glucose-binding protein, preferably glucose oxidase.

**4.** An optical biosensor according to any preceding claim, wherein said analyte-binding protein is covalently conjugated to said SWCNT *via* a linker.

**5.** An optical biosensor according to claim 4, wherein the optical biosensor has the structure:

SWCNT- X - p

wherein

SWCNT is a single-walled carbon nanotube;
X is a linker;
p is an analyte-binding protein; and
- are covalent bonds.

**6.** An optical biosensor according to any one of claims 4 to 5, wherein said linker is at an sp3 defect on the SWCNT.

**7.** An optical biosensor according to any one of claims 4 to 6, wherein the linker comprises a group selected from the following:

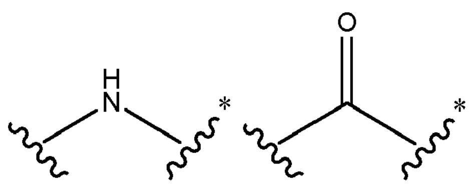

wherein wavy bonds marked by a * denote a preferred point of attachment to the analyte-binding protein.

8. An optical biosensor according to claim 7, wherein the linker comprises a group selected from the following:

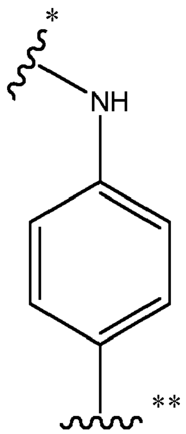 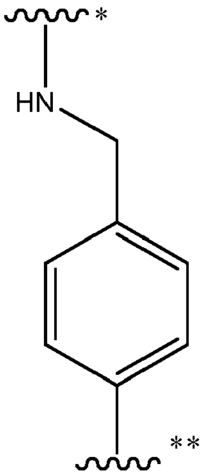 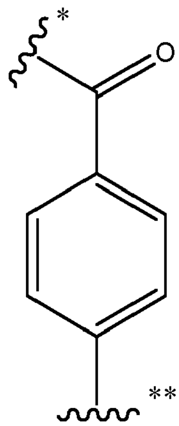

wherein the aryl rings are optionally substituted, wavy bonds marked by a * denote a preferred point of attachment to the analyte-binding protein, and wavy bonds marked by a ** denote a preferred point of attachment to the SWCNT.

9. An optical biosensor according to any one of claims 4 to 8, wherein the analyte-binding protein and the linker are covalently linked *via* a carbonyl-containing bond, preferably an ester, an amide, or a thioester bond, more preferably an amide bond.

10. An optical biosensor according to any preceding claim, wherein said optical biosensor exhibits a change in optical properties, preferably a change in fluorescence properties, when binding said analyte.

11. A method of preparing an optical biosensor according to any preceding claim, the method comprising covalently conjugating an analyte-binding protein to an SWCNT.

12. Use of an optical biosensor according to any one of claims 1 to 10 as a biosensor for an analyte, preferably a sugar, more preferably glucose.

13. Use of an optical biosensor according to any one of claims 1 to 10 in the monitoring, detection, and/or quantification of an analyte, preferably a sugar, more preferably glucose, in a sample.

14. A method of monitoring, quantifying, and/or detecting an analyte, preferably a sugar, more preferably glucose, in a sample, the method comprising:

contacting an optical biosensor according to any one of claims 1 to 10 to a sample comprising an analyte;
monitoring a change in optical properties of the conjugate; and optionally
correlating said change with an analyte level.

15. A composition, preferably a suspension, comprising an optical biosensor according to any one of claims 1 to 10, a solvent, and, optionally, an analyte, preferably a sugar, more preferably glucose.

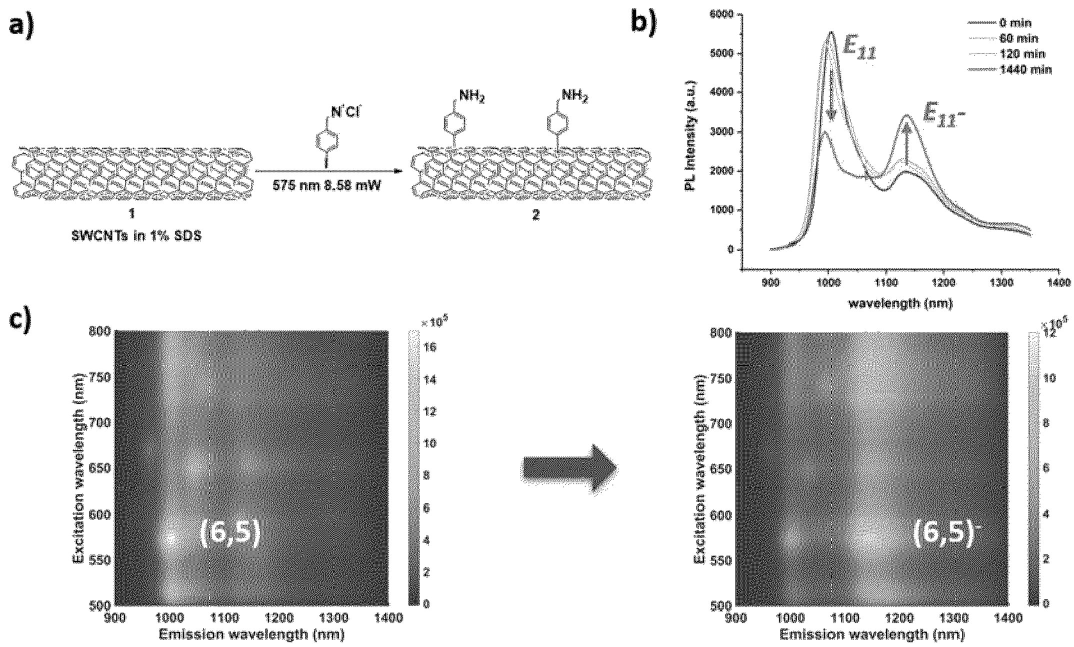
a)
575 nm 8.58 mW
1
2
SWCNTs in 1% SDS
b)
0 min
60 min
120 min
1440 min
PL Intensity (a.u.)
wavelength (nm)
c)
Excitation wavelength (nm)
Emission wavelength (nm)
(6,5)
(6,5)-

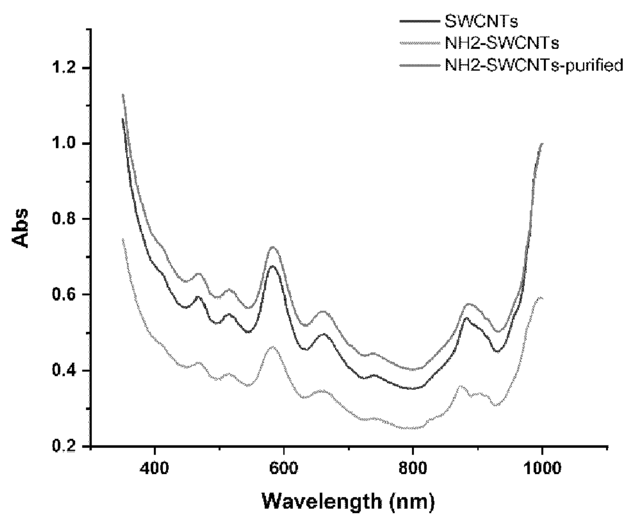
SWCNTs
NH2-SWCNTs
NH2-SWCNTs-purified
Abs
Wavelength (nm)

d)

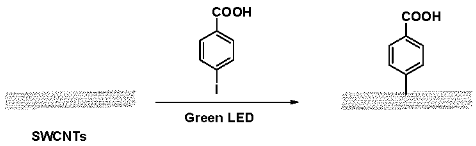


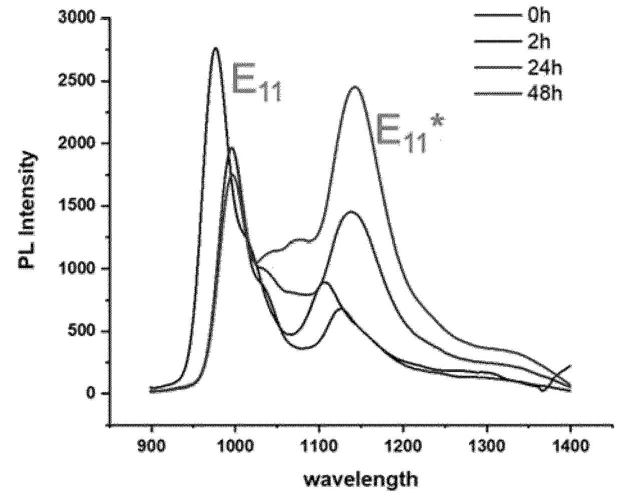


(e)

Figure 1(a)-(e)

a)

| Entry | Coupling reagent | Solvent | Result |
|---|---|---|---|
| 1 | EDC | Water | Difficult for monitoring |
| 2 | EDC/NHS | Tris buffer | No reaction |
| 3 | EDC/NHS | MES buffer | No reaction |
| 4 | EDC/NHS | Water/DMSO | Good reaction |
| 5 | DMTMM | MES buffer | No reaction |

b)

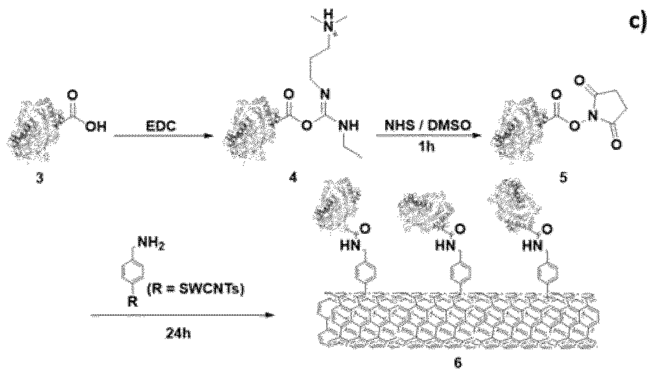


3 4 5 6

c)

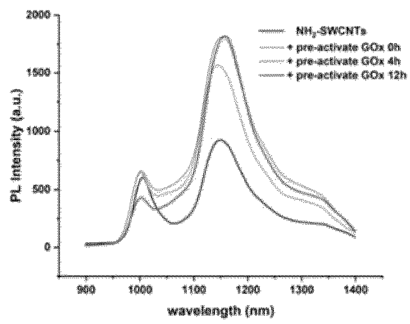

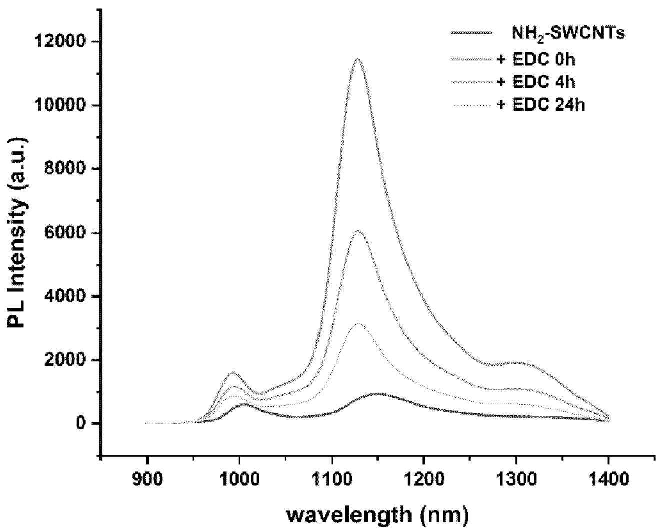


(d)

Figures 2(a)-(d)

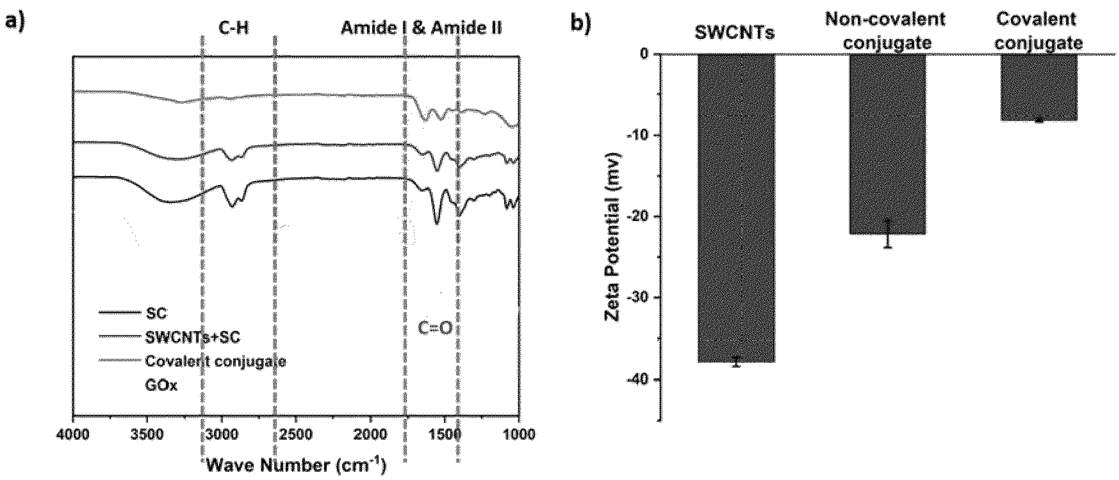


Figures 3(a) and 3(b)

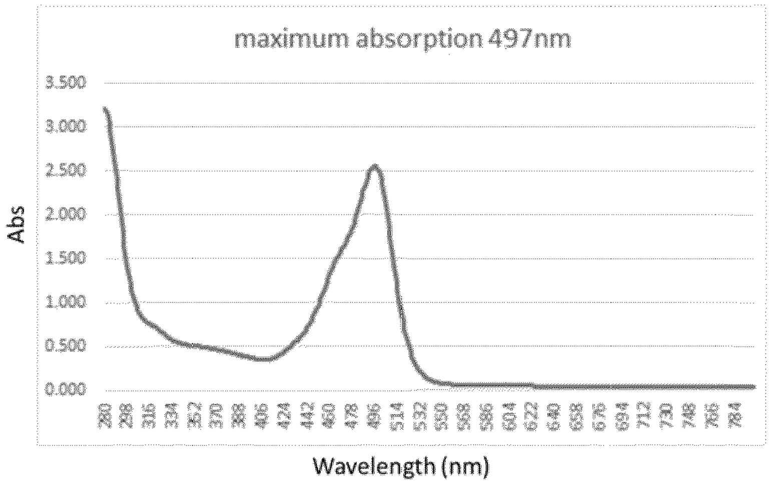


Figure 4

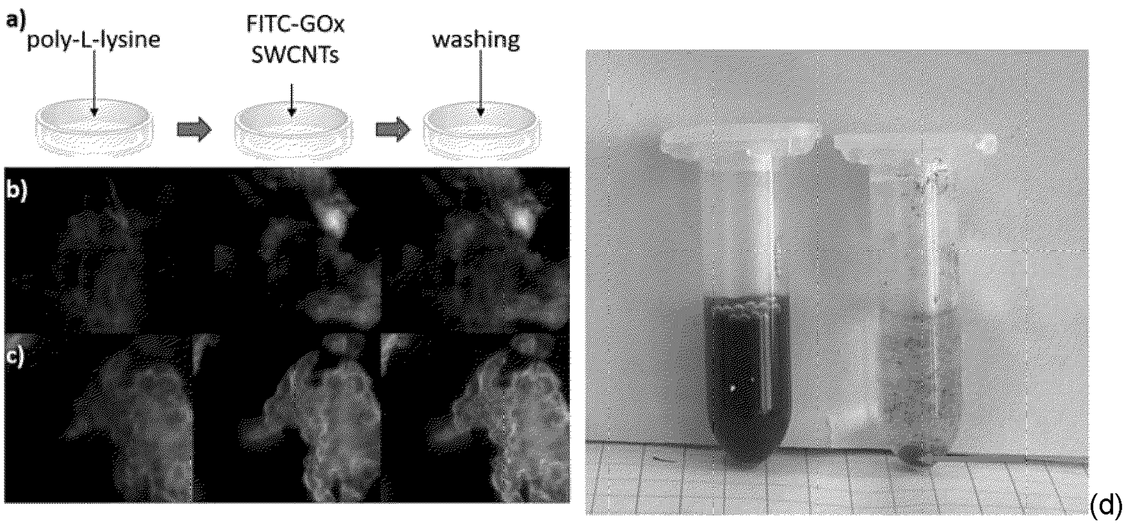


Figures 5(a)-(d)

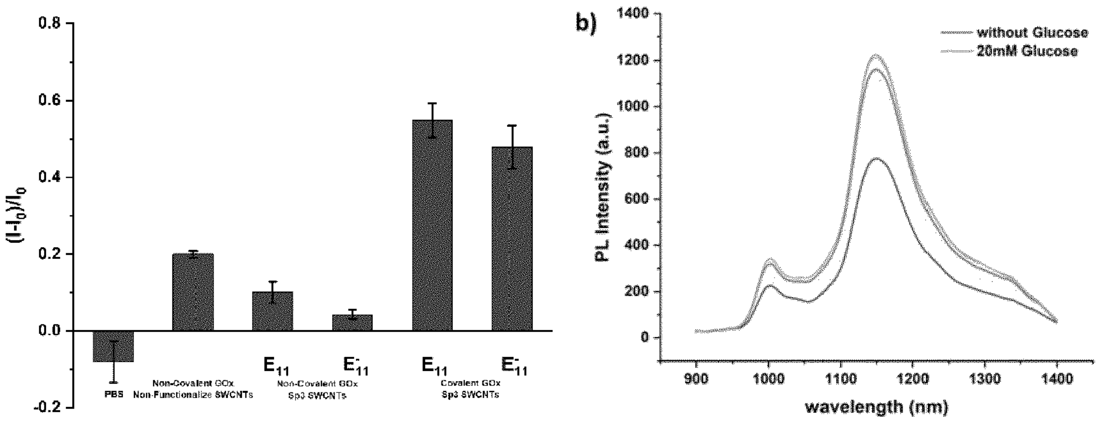


Figures 6(a) and (b)

Europäisches Patentamt
European Patent Office
Office européen des brevets

# EUROPEAN SEARCH REPORT

Application Number
EP 24 16 2781

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | ZUBKOVS VITALIJS ET AL: "Bioengineering a glucose oxidase nanosensor for near-infrared continuous glucose monitoring", NANOSCALE ADVANCES, vol. 4, no. 11, 1 January 2022 (2022-01-01), pages 2420-2427, XP093191451, ISSN: 2516-0230, DOI: 10.1039/D2NA00092J * the whole document * * in particular: * * abstract * * section "Bioconjugation of GOx with thiol-reactive crosslinkers"; page 2421 - page 2423 * * section "Glucose detection using the GOx(70C)-PM-SWCNT sensor"; page 2423 * ----- | 1-15 | INV. C12Q1/00 C12Q1/26 B82Y15/00 |
| Y | XUE HUAIGUO ET AL: "Single-wall carbon manotubes as immobilization material for glucose biosensor", SYNTHETIC METALS, vol. 135-136, 1 April 2003 (2003-04-01), pages 831-832, XP093191633, CH ISSN: 0379-6779, DOI: 10.1016/S0379-6779(02)00919-0 * the whole document * * in particular: * * abstract * ----- | 1-15 | TECHNICAL FIELDS SEARCHED (IPC) C12Q B82Y |

-/--

The present search report has been drawn up for all claims

1

| Place of search | Date of completion of the search | Examiner |
|---|---|---|
| The Hague | 1 August 2024 | Tuynman, Antonin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 1 of 5

Europäisches Patentamt
European Patent Office
Office européen des brevets

# EUROPEAN SEARCH REPORT

Application Number
EP 24 16 2781

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | FLORIAN A MANN ET AL: "Quantum Defects as a Toolbox for the Covalent Functionalization of Carbon Nanotubes with Peptides and Proteins", ANGEWANDTE CHEMIE INTERNATIONAL EDITION, VERLAG CHEMIE, HOBOKEN, USA, vol. 59, no. 40, 13 July 2020 (2020-07-13), pages 17732-17738, XP072096126, ISSN: 1433-7851, DOI: 10.1002/ANIE.202003825<br>* the whole document *<br>* in particular: *<br>* abstract *<br>* figures 1,3 *<br>* page 17736, left-hand column, line 27 - line 30 *<br>* page 17735, left-hand column, line 5 - line 11 *<br>----- | 1-15 | |
| Y | MANN FLORIAN A ET AL: "Quantum defects as versatile anchors for carbon nanotube functionalization", NATURE PROTOCOLS, NATURE PUBLISHING GROUP, GB, vol. 17, no. 3, 2 February 2022 (2022-02-02), pages 727-747, XP037713095, ISSN: 1754-2189, DOI: 10.1038/S41596-021-00663-6 [retrieved on 2022-02-02]<br>* the whole document *<br>* in particular: *<br>* abstract *<br>* section "Applications of the Method"; page 729 *<br>* figure 2 *<br>-----<br>-/-- | 1-15 | TECHNICAL FIELDS SEARCHED (IPC) |

The present search report has been drawn up for all claims

1

| Place of search | Date of completion of the search | Examiner |
|---|---|---|
| The Hague | 1 August 2024 | Tuynman, Antonin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 2 of 5

# EUROPEAN SEARCH REPORT

Application Number
EP 24 16 2781

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | SUMA YANASINEE ET AL: "Noncovalent and covalent immobilization of oxygenase on single-walled carbon nanotube for enzymatic decomposition of aromatic hydrocarbon intermediates", ENVIRONMENTAL SCIENCE AND POLLUTION RESEARCH, SPRINGER BERLIN HEIDELBERG, BERLIN/HEIDELBERG, vol. 23, no. 2, 7 February 2015 (2015-02-07), pages 1015-1024, XP035937797, ISSN: 0944-1344, DOI: 10.1007/S11356-015-4168-5 [retrieved on 2015-02-07] * the whole document * * in particular: * * abstract * * figure 1 * ----- | 1,4-15 | |
| Y | JUDITH H BARTHA-VÁRI ET AL: "Immobilization of Phenylalanine Ammonia-Lyase on Single-Walled Carbon Nanotubes for Stereoselective Biotransformations in Batch and Continuous-Flow Modes", CHEMCATCHEM, JOHN WILEY & SONS, INC, HOBOKEN, USA, vol. 7, no. 7, 12 March 2015 (2015-03-12), pages 1122-1128, XP072434693, ISSN: 1867-3880, DOI: 10.1002/CCTC.201402894 * the whole document * * in particular: * * abstract * * Scheme 1; page 1123 * * section "Conclusion"; page 1126 * ----- -/-- | 1,4-15 | TECHNICAL FIELDS SEARCHED (IPC) |

The present search report has been drawn up for all claims

1

| Place of search | Date of completion of the search | Examiner |
|---|---|---|
| The Hague | 1 August 2024 | Tuynman, Antonin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 24 16 2781

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | BENCZE LÁSZLÓ CSABA ET AL: "Nanobioconjugates ofCandida antarcticalipase B and single-walled carbon nanotubes in biodiesel production", BIORESOURCE TECHNOLOGY, ELSEVIER, AMSTERDAM, NL, vol. 200, 27 October 2015 (2015-10-27), pages 853-860, XP029312823, ISSN: 0960-8524, DOI: 10.1016/J.BIORTECH.2015.10.072 * the whole document * * in particular: * * abstract * ----- | 1,4-15 | |
| Y | BOROS KRISZTINA ET AL: "Robust, site-specifically immobilized phenylalanine ammonia-lyases for the enantioselective ammonia addition of cinnamic acids", CATALYSIS SCIENCE & TECHNOLOGY, vol. 11, no. 16, 1 January 2021 (2021-01-01), pages 5553-5563, XP093191640, UK ISSN: 2044-4753, DOI: 10.1039/D1CY00195G * the whole document * * in particular: * * abstract * ----- | 1,4-15 | TECHNICAL FIELDS SEARCHED (IPC) |

-/--

The present search report has been drawn up for all claims

1

| Place of search | Date of completion of the search | Examiner |
|---|---|---|
| The Hague | 1 August 2024 | Tuynman, Antonin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 24 16 2781

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | THOMAS SUZANNE K. ET AL: "Site-Specific Protein Photochemical Covalent Attachment to Carbon Nanotube Side Walls and Its Electronic Impact on Single Molecule Function", BIOCONJUGATE CHEMISTRY, vol. 31, no. 3, 19 November 2019 (2019-11-19), pages 584-594, XP093191625, US ISSN: 1043-1802, DOI: 10.1021/acs.bioconjchem.9b00719 * the whole document * * in particular: * * abstract * * figures 1,4 * ----- | 1,4-15 | |
| Y | WANG HANXUAN ET AL: "Covalent conjugation of proteins onto fluorescent single-walled carbon nanotubes for biological and medical applications", MATERIALS ADVANCES, vol. 4, no. 3, 29 November 2022 (2022-11-29), pages 823-834, XP093191492, ISSN: 2633-5409, DOI: 10.1039/D2MA00714B * the whole document * ----- | 1-15 | TECHNICAL FIELDS SEARCHED (IPC) |

The present search report has been drawn up for all claims

1

| Place of search | Date of completion of the search | Examiner |
|---|---|---|
| The Hague | 1 August 2024 | Tuynman, Antonin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2020254336 A

### Non-patent literature cited in the description


- **J. YANG** ; **Z. ZHANG** ; **D. ZHANG** ; **Y. LI**. *Materials Chemistry and Physics*, 2013, vol. 139, 233-240
- **ZUBKOVS, V. et al.** *Small*, 2017, vol. 13, 1701654
- **O. SCHNELL** ; **E. STANDI**. *Endocrine Practice*, 2006, vol. 12, 16-19
- **S. DEWANJEE** ; **P. CHAKRABORTY** ; **H. BHATTACHARYA** ; **L. CHACKO** ; **B. SINGH** ; **A. CHAUDHARY** ; **K. JAVVAJI** ; **S. R. PRADHAN** ; **J. VALLAMKONDU** ; **A. DEY**. *Free Radical Biology and Medicine*, 2022, vol. 193, 134-157
- **A. L. GALANT** ; **R. C. KAUFMAN** ; **J. D. WILSON**. *Food Chemistry*, 2015, vol. 188, 149-160
- **M.-S. STEINER** ; **A. DUERKOP** ; **O. S. WOLFBEIS**. *Chemical Society Reviews*, 2011, vol. 40, 4805-4839
- **E. S. FORZANI** ; **H. ZHANG** ; **L. A. NAGAHARA** ; **I. AMLANI** ; **R. TSUI** ; **N. TAO**. *Nano Letters*, 2004, vol. 4, 1785-1788
- **L. OLANSKY** ; **L. KENNEDY**. *Diabetes Care*, 2010, vol. 33, 948-949
- **M. EL KHOURY** ; **F. YOUSUF** ; **V. MARTIN** ; **R. M. COHEN**. *Endocrine Practice*, 2008, vol. 14, 337-339
- **T. V. GALASSI** ; **M. ANTMAN-PASSIG** ; **Z. YAARI** ; **J. JESSURUN** ; **R. E. SCHWARTZ** ; **D. A. HELLER**. *PLOS ONE*, 2020, vol. 15, e0226791
- **D. MEYER** ; **S. TELELE** ; **A. ZELENÁ** ; **A. J. GILLEN** ; **A. ANTONUCCI** ; **E. NEUBERT** ; **R. NIßLER** ; **F. A. MANN** ; **L. ERPENBECK** ; **A. A. BOGHOSSIAN**. *Nanoscale*, 2020, vol. 12, 9104-9115
- **J. XU** ; **R. MUELLER** ; **E. HAZELBAKER** ; **Y. ZHAO** ; **J.-C. J. BONZONGO** ; **J. G. CLAR** ; **S. VASENKOV** ; **K. J. ZIEGLER**. *Langmuir*, 2017, vol. 33, 5006-5014
- **R. HAGGENMUELLER** ; **S. S. RAHATEKAR** ; **J. A. FAGAN** ; **J. CHUN** ; **M. L. BECKER** ; **R. R. NAIK** ; **T. KRAUSS** ; **L. CARLSON** ; **J. F. KADLA** ; **P. C. TRULOVE**. *Langmuir*, 2008, vol. 24, 5070-5078
- **S. KRUSS** ; **M. P. LANDRY** ; **E. VANDER ENDE** ; **B. M. A. LIMA** ; **N. F. REUEL** ; **J. ZHANG** ; **J. NELSON** ; **B. MU** ; **A. HILMER** ; **M. STRANO**. *Journal of the American Chemical Society*, 2014, vol. 136, 713-724
- **R. NIßLER** ; **L. KURTH** ; **H. LI** ; **A. SPREINAT** ; **I. KUHLEMANN** ; **B. S. FLAVEL** ; **S. KRUSS**. *Analytical Chemistry*, 2021, vol. 93, 6446-6455
- **X. ZHANG** ; **L. MENG** ; **Q. LU**. *ACS Nano*, 2009, vol. 3, 3200-3206
- **Y. YOMOGIDA** ; **T. TANAKA** ; **M. TSUZUKI** ; **X. WEI** ; **H. KATAURA**. *ACS Applied Nano Materials*, 2020, vol. 3, 11289-11297
- **A. HENDLER-NEUMARK** ; **V. WULF** ; **G. BISKER**. *ACS Sensors*, 2023, vol. 8, 3713-3722
- **C. FARRERA** ; **F. TORRES ANDÓN** ; **N. FELIU**. *ACS Nano*, 2017, vol. 11, 10637-10643
- **M. LASKOWSKI** ; **M. A. QASIM**. *Biochimica et Biophysica Acta (BBA) - Protein Structure and Molecular Enzymology*, 2000, vol. 1477, 324-337
- **L. J. C. JEUKEN** ; **A. K. JONES** ; **S. K. CHAPMAN** ; **G. CECCHINI** ; **F. A. ARMSTRONG**. *Journal of the American Chemical Society*, 2002, vol. 124, 5702-5713
- **C. BERGERET** ; **J. COUSSEAU** ; **V. FERNANDEZ** ; **J.-Y. MEVELLEC** ; **S. LEFRANT**. *The Journal of Physical Chemistry C*, 2008, vol. 112, 16411-16416
- **H. WANG** ; **A. A. BOGHOSSIAN**. *Materials Advances*, 2023, vol. 4, 823-834
- **Y. PIAO** ; **B. MEANY** ; **L. R. POWELL** ; **N. VALLEY** ; **H. KWON** ; **G. C. SCHATZ** ; **Y. WANG**. *Nature Chemistry*, 2013, vol. 5, 840-845
- **T. SHIRAKI** ; **Y. MIYAUCHI** ; **K. MATSUDA** ; **N. NAKASHIMA**. *Accounts of Chemical Research*, 2020, vol. 53, 1846-1859
- **J. DE MEUTTER** ; **E. GOORMAGHTIGH**. *Analytical Chemistry*, 2021, vol. 93, 3733-3741
- **X. ZHAO** ; **D. LU** ; **F. HAO** ; **R. LIU**. *Journal of Hazardous Materials*, 2015, vol. 292, 98-107

- **F. A. MANN** ; **N. HERRMANN** ; **F. OPAZO** ; **S. KRUSS**. *Angew. Chem. Int. Ed.*, 2020, vol. 59, 17732